# EUROPEAN PATENT SPECIFICATION

(11) **EP 3 914 701 B1**
(45) Date of publication and mention of the grant of the patent: **03.12.2025**
(21) Application number: 20744495.1
(22) Date of filing: 23.01.2020
(51) Int. Cl.: A61K 31/277, A61K 31/4015, A61K 31/437, A61K 31/44, A61K 31/522, A61K 31/53, A61K 31/69, A61K 31/7105, C12N 9/16, C12Q 1/44, A61P 25/08

(54) **METHODS OF TREATING EPILEPSY VIA PHOSPHODIESTERASE 4 B (PDE4) INHIBITION**
VERFAHREN ZUR BEHANDLUNG VON EPILEPSIE ÜBER PHOSPHODIESTERASE 4-(PDE4) B HEMMUNG
MÉTHODES DE TRAITEMENT DE L'ÉPILEPSIE PAR INHIBITION DE LA PHOSPHODIESTÉRASE 4 B (PDE4)

(30) Priority: 23.01.2019 US 201962796002 P
(43) Date of publication of application: 01.12.2021
(73) Proprietor: Path Therapeutics Inc., Calgary, AB T2N 3Z4 (CA)
(72) Inventor: IBHAZEHIEBO, Kingsley, Calgary, Alberta T23 3W5 (CA); KURRASCH, Deborah M., Calgary, Alberta T2N 3Z4 (CA)
(74) Representative: Boult Wade Tennant LLP
(86) International application number: PCT/US2020/014819
(87) International publication number: WO 2020/154520

(56) References cited:
- WO-A1-2017/145013
- US-A1- 2005 164 275
- US-A1- 2006 106 085
- US-A1- 2009 186 896
- US-A1- 2011 230 504
- US-A1- 2013 065 936
- US-A1- 2013 178 498
- US-A1- 2014 349 969
- US-A1- 2015 232 857
- ESKANDARI NAHID ET AL: "A short review on structure and role of cyclic-3',5'-adenosine monophosphate-specific phosphodiesterase 4 as a treatment tool", JOURNAL OF RESEARCH IN PHARMACY PRACTICE, vol. 4, no. 4, 1 January 2015 (2015-01-01), pages 175, XP055957735, ISSN: 2279-042X, DOI: 10.4103/2279-042X.167043
- ENDO PHARMACEUTICALS INC: "Indications & Usage Dosage & Administration Contraindications Precautions Warnings Adverse Reactions Drug Interactions Overdosage Description Clinical Pharmacology Clinical Studies How Supplied/Storage And Handling Storage And Handling Patient Counseling Information Print Version Drug Label Sections", MG -FD&C RED NO. 40 AND D&C RED NO. 28, 1 August 2017 (2017-08-01), pages 24, XP055957350, Retrieved from the Internet <URL:https://www.endo.com/File%20Library/Products/Prescribing%20Information/Theo-24_prescribing_information.html> [retrieved on 20220902]
- ZHANG CHONG ET AL: "Memory enhancing effects of BPN14770, an allosteric inhibitor of phosphodiesterase-4D, in wild-type and humanized mice", NEUROPSYCHOPHARMACOLOGY, vol. 43, no. 11, 14 August 2018 (2018-08-14), Cham, pages 2299 - 2309, XP093068992, ISSN: 0893-133X, Retrieved from the Internet <URL:http://www.nature.com/articles/s41386-018-0178-6.pdf> DOI: 10.1038/s41386-018-0178-6
- ALEX B BURGIN ET AL: "Design of phosphodiesterase 4D (PDE4D) allosteric modulators for enhancing cognition with improved safety", NATURE BIOTECHNOLOGY, vol. 28, no. 1, 1 January 2010 (2010-01-01), pages 63 - 70, XP055060047, ISSN: 1087-0156, DOI: 10.1038/nbt.1598

## Description

### INTRODUCTION

Tens of millions of people suffer from epilepsy. Despite nearly eight decades of research and the advent of many new drugs, the efficacy rates for seizure relief have not significantly changed. Drugs are the mainstay treatment for epilepsy but a stubborn 30-40% of epileptic patients are refractory to current medications and will have unremitting recurrent seizures and attendant life-long health problems. By way of example, Dravet syndrome (DS) is a childhood epilepsy that usually appears in the first year of life in an otherwise healthy baby as a febrile seizure lasting more than five minutes (often longer than 30 minutes). Most cases of DS are due to loss-of-function mutations in the *Scn1a* gene encoding brain voltage-gated sodium channel type-I, Na_{V}1.1. Despite its genetics being understood, DS remains highly pharmacoresistant and thousands of children struggle through numerous ineffective therapies.

Following DS diagnosis, families are shuffled in and out of clinics and ERs with the kids having repeated EEGs, CTs, MRIs, and spinal taps as clinicians try to understand the disease and assess treatment strategies. This means young children (and their families) are put through the gruelling task of trying various primarily adult antiseizure therapies singly and in combination (n=8 drugs and/or surgery and/or the ketogenic diet) in the hopes of finding an efficacious strategy. Given that it can take 8-10 weeks to test one strategy, only 5-6 combinations might be tested in a year and multiple years are often required to identify a treatment strategy - and all the while the children continue to have uncontrolled seizures and visits to the emergency room.

In June 2018, the FDA approved cannabidiol (CBD; Epidiolex) as the first antiseizure medication for DS. While an important step forward, CBD only reduces seizure frequency in 43% of patients and fewer than 5% achieved full seizure freedom (the ultimate goal). Thus, there remains a significant unmet need for efficacious anti-epileptic agents.

US 2011/0230504 A1 relates to compositions and methods for promoting and enhancing the analgesic, anesthetic and anticonvulsant properties of epoxygenated fatty acids, in particular, epoxy-eicosatrienoic acids ("EETs") and inhibitors of soluble epoxide hydrolase ("sEH") in the presence of elevated levels of cyclic adenosine monophosphate ("cAMP") by combining or co-administering the epoxygenated fatty acid, EET and/or inhibitor of sEH with an agent that increases intracellular levels of cAMP, e.g., a phosphodiesterase inhibitor.

### SUMMARY

The invention provides a phosphodiesterase 4 (PDE4) inhibitor for use in a method of treating epilepsy, wherein the method comprises administering to an individual having epilepsy a therapeutically effective amount of the PDE4 inhibitor, and wherein the PDE4 inhibitor is selective for PDE4B.

### BRIEF DESCRIPTION OF THE FIGURES

**FIG. 1****:** An overview of a representative PDE4 gene structure.
**FIG. 2****:** Amino acid sequence alignment of human PDE4A polypeptides that may be inhibited according to embodiments of the present disclosure.
**FIG. 3****:** Amino acid sequence alignment of human PDE4B polypeptides that may be inhibited according to embodiments of the present disclosure.
**FIG. 4****:** Amino acid sequence alignment of human PDE4C polypeptides that may be inhibited according to embodiments of the present disclosure.
**FIG. 5****:** Amino acid sequence alignment of human PDE4D polypeptides that may be inhibited according to embodiments of the present disclosure.
**FIG. 6****:** Panel A: data demonstrating that an example PDE4 inhibitor (AN2728) dose-dependently restores bioenergetics to baseline levels in *scn1lab* mutant zebrafish. Panel B: data demonstrating that a variety of PDE4 inhibitors (in this example, Rolipram, Cilomilast, Roflumilast, Ibudilast, Theophylline, Drotaverine, and Irsogladine) are effective in restoring bioenergetics to baseline levels in *scn1lab* mutant zebrafish.
**FIG. 7****:** Data demonstrating that PDE4B-, 4C- and 4D-morpholinos are effective in restoring bioenergetics to baseline levels in *scn1lab* mutant zebrafish.
**FIG 8****:** Data demonstrating that two examples of PDE4 inhibitors (Roflumilast and Theophylline) restore bioenergetics in a *kcna1* model of generalizable epilepsy.
**FIG. 9****:** Panels A and B: Data demonstrating that an example PDE4 inhibitor (AN2728) blocks seizure-like hyperexcitability to baseline levels in *scn1lab* zebrafish, including decreases in seizure frequency and peak amplitude.
**FIG. 10****:** Panels A and B: Data demonstrating that a PDE4B morpholino is effective in blocking seizure-like hyperexcitability to baseline levels in *scn1lab* zebrafish.
**FIG. 11****:** Data demonstrating that an example PDE4 inhibitor (AN2728) decreases hyperexcitability in mouse *Scn1a* mutant brain *ex vivo* slices.
**FIG. 12****:** Data demonstrating that an example PDE4 inhibitor (AN2728) protects against induction of seizures in a 6Hz-induction mouse model.
**FIG. 13****:** Data demonstrating that an example PDE4 inhibitor (AN2728) protects against hyperthermia-induced seizures in *Scn1a* mutant mice.
**FIG. 14****:** Data demonstrating that a two-fold selective PDE4B inhibitor (rolipram) is effective in protecting against hyperthermia-induced seizures in *Scn1a* mutant mice.
**FIG 15****:** Data demonstrating that three examples of PDE4 inhibitors (AN2728, Rolipram, Roflumilast) partially or fully block seizures in *Scn1a* mutant mice.

### DETAILED DESCRIPTION

The invention provides a phosphodiesterase 4 (PDE4) inhibitor for use in a method of treating epilepsy, wherein the method comprises administering to an individual having epilepsy a therapeutically effective amount of the PDE4 inhibitor, and wherein the PDE4 inhibitor is selective for PDE4B.

All aspects of the invention relate to a phosphodiesterase 4 (PDE4) inhibitor which is selective for PDE4B. Thus, aspects herein regarding a PDE4 inhibitor of the invention relate to a PDE4B which is selective for PDE4B. The disclosure herein should be read with this in mind.

Methods of treatment of the human body by therapy, referred to in this description, are not part of the present invention as such, but are described here in relation to compounds and pharmaceutical compositions for use in said methods for treatment of the human body by therapy, according to the present invention.

Where a range of values is provided, it is understood that each intervening value, to the tenth of the unit of the lower limit unless the context clearly dictates otherwise, between the upper and lower limit of that range and any other stated or intervening value in that stated range, is encompassed within the methods. The upper and lower limits of these smaller ranges may independently be included in the smaller ranges and are also encompassed within the methods, subject to any specifically excluded limit in the stated range. Where the stated range includes one or both of the limits, ranges excluding either or both of those included limits are also included in the methods.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which the methods belong. Although any methods similar or equivalent to those described herein can also be used in the practice or testing of the methods, representative illustrative methods are now described.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It is appreciated that certain features of the methods, which are, for clarity, described in the context of separate embodiments, may also be provided in combination in a single embodiment. Conversely, various features of the methods, which are, for brevity, described in the context of a single embodiment, may also be provided separately or in any suitable sub-combination. All combinations of the embodiments are specifically embraced by the present disclosure and are disclosed herein just as if each and every combination was individually and explicitly disclosed, to the extent that such combinations embrace operable processes and/or compositions. In addition, all sub-combinations listed in the embodiments describing such variables are also specifically embraced by the present methods and are disclosed herein just as if each and every such sub-combination was individually and explicitly disclosed herein.

As will be apparent to those of skill in the art upon reading this disclosure, each of the individual embodiments described and illustrated herein has discrete components and features which may be readily separated from or combined with the features of any of the other several embodiments. Any recited method can be carried out in the order of events recited or in any other order that is logically possible.

### METHODS OF TREATMENT

As summarized above, the invention provides a phosphodiesterase 4 (PDE4) inhibitor for use in a method of treating epilepsy, wherein the method comprises administering to an individual having epilepsy a therapeutically effective amount of the PDE4 inhibitor, and wherein the PDE4 inhibitor is selective for PDE4B.. The methods are based in part on the unexpected findings described herein that PDE4 inhibition: blocks a bioenergetics and hyperexcitable neuronal phenotype in both zebrafish and mouse models of epilepsy; protects against hyperthermia-induced seizures in a mouse model of epilepsy; and blocks seizures using a 6Hz test - the entry-point model of the NIH-backed Epilepsy Therapy Screening Program.

Cyclic nucleotide phosphodiesterases (PDEs) catalyze the hydrolysis of the cyclic nucleotide second messengers - cyclic adenosine monophosphate (cAMP) and cyclic guanosine monophosphate (cGMP). The PDE4 family is one of three cAMP-specific PDE families. PDE4s have been shown to regulate several cellular physiological processes, including protein phosphorylation via cAMP-dependent protein kinase A (PKA), gene transcription through cAMP response elements, and cyclic nucleotide gated ion channels. These processes have been linked to cognitive function, depression, schizophrenia, hypertension, and cardiomyocyte contractility.

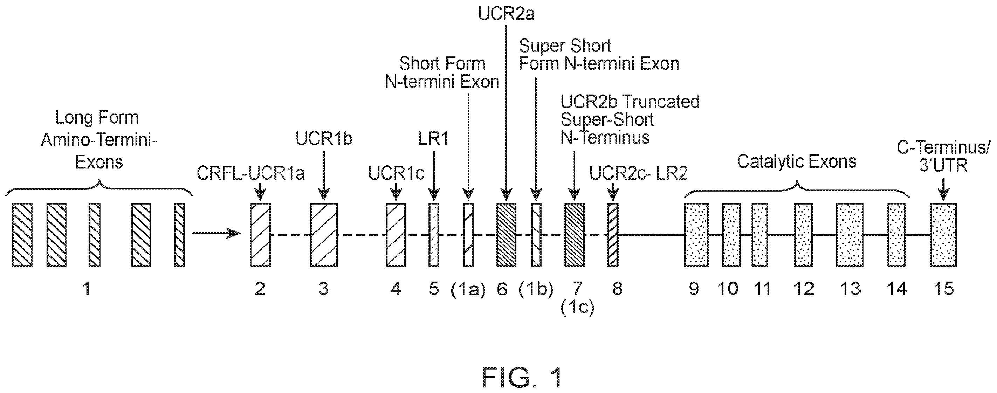

The PDE4 gene family is composed of four gene isoforms: PDE4A, PDE4B, PDE4C, and PDE4D. The isoforms arose via a gene duplication event in a common eukaryotic ancestor before the separation of sponges and eumetazoans. Transcripts from all four PDE4 gene isoforms have been detected in mammalian species. An overview of a representative PDE4 gene structure is provided in FIG. 1. PDE4 genes are composed of multiple exons connected by either a dashed line (facultative exons), or solid line (constitutive exons). The PDE4 long form amino-termini specifying exons (1), are located in the upstream region of the genes, each under different promoter control. The upstream conserved region-1 (UCR1) is composed of three exons (UCR1a, UCR1b, and UCR1c). The PDE4 short form amino termini specifying exon(s) (1a) are located downstream of the linker region 1 (LR1) exon. Upstream conserved region 2 (UCR2) is composed of three exons (UCR2a, UCR2b and UCR2c), which are interrupted by the super-short form amino-terminus specifying exon (1b). The amino-terminus of truncated super-short PDE4 splice variants is found within the UCR2b exon (1c). The enzymatic core of PDE4 is encoded by several constitutive exons (found in all isoforms) located in the farthest downstream regions of the gene. Further details regarding the gene structure and splice variants of the PDE4 isoforms are found, e.g., in Johnson et al. (2010) BMC Evol Biol. 10:247. In some embodiments, a PDE4 polypeptide inhibited according to the methods of the present disclosure is a PDE4 polypeptide provided in FIG. S1 of Johnson et al. (2010) BMC Evol Biol. 10:247.

Non-limiting examples of PDE4 polypeptides which may be inhibited (alone or in any combination)according to the methods of the present disclosure are provided in Table 1.

**Table 1 - Example Human PDE4 target polypeptides**

| | |
|---|---|
| PDE4A (Human Isoform 1) | MEPPTVPSERSLSLSLPGPREGQATLKPPPQHLWRQPRTPIRIQQRGYS DSAERAERERQPHRPIERADAMDTSDRPGLRTTRMSWPSSFHGTGTGS GGAGGGSSRRFEAENGPTPSPGRSPLDSQASPGLVLHAGAATSQRRES FLYRSDSDYDMSPKTMSRNSSVTSEAHAEDLIVTPFAQVLASLRSVRSNF SLLTNVPVPSNKRSPLGGPTPVCKATLSEETCQQLARETLEELDWCLEQL ETMQTYRSVSEMASHKFKRMLNRELTHLSEMSRSGNQVSEYISTTFLDK QNEVEIPSPTMKEREKQQAPRPRPSQPPPPPVPHLQPMSQITGLKKLMH SNSLNNSNIPRFGVKTDQEELLAQELENLNKWGLNIFCVSDYAGGRSLTC IMYMIFQERDLLKKFRIPVDTMVTYMLTLEDHYHADVAYHNSLHAADVLQ STHVLLATPALDAVFTDLEILAALFAAAIHDVDHPGVSNQFLINTNSELALM YNDESVLENHHLAVGFKLLQEDNCDIFQNLSKRQRQSLRKMVIDMVLATD |
| UniProtKB - P27815-1) | |
| SEQ ID NO:1 | |
| | MSKHMTLLADLKTMVETKKVTSSGVLLLDNYSDRIQVLRNMVHCADLSN PTKPLELYRQWTDRIMAEFFQQGDRERERGMEISPMCDKHTASVEKSQ VGFIDYIVHPLWETWADLVHPDAQEILDTLEDNRDWYYSAIRQSPSPPPE EESRGPGHPPLPDKFQFELTLEEEEEEEISMAQIPCTAQEALTAQGLSGV EEALDATIAWEASPAQESLEVMAQEASLEAELEAVYLTQQAQSTGSAPV APDEFSSREEFVVAVSHSSPSALALQSPLLPAWRTLSVSEHAPGLPGLPS TAAEVEAQREHQAAKRACSACAGTFGEDTSALPAPGGGGSGGDPT |
| PDE4A (Human Isoform 2) | MARPRGLGRIPELQLVAFPVAVAAEDEAFLPEPLAPRAPRRPRSPPSSPV FFASPSPTFRRRLRLLRSCQDLGRQAWAGAGFEAENGPTPSPGRSPLD SQASPGLVLHAGAATSQRRESFLYRSDSDYDMSPKTMSRNSSVTSEAH AEDLIVTPFAQVLASLRSVRSNFSLLTNVPVPSNKRSPLGGPTPVCKATLS EETCQQLARETLEELDWCLEQLETMQTYRSVSEMASHKFKRMLNRELTH LSEMSRSGNQVSEYISTTFLDKQNEVEIPSPTMKEREKQQAPRPRPSQP PPPPVPHLQPMSQITGLKKLMHSNSLNNSNIPRFGVKTDQEELLAQELEN LNKWGLNIFCVSDYAGGRSLTCIMYMIFQERDLLKKFRIPVDTMVTYMLTL EDHYHADVAYHNSLHAADVLQSTHVLLATPALDAVFTDLEILAALFAAAIH DVDHPGVSNQFLINTNSELALMYNDESVLENHHLAVGFKLLQEDNCDIFQ NLSKRQRQSLRKMVIDMVLATDMSKHMTLLADLKTMVETKKVTSSGVLLL DNYSDRIQVLRNMVHCADLSNPTKPLELYRQWTDRIMAEFFQQGDRERE RGMEISPMCDKHTASVEKSQVGFIDYIVHPLWETWADLVHPDAQEILDTL EDNRDWYYSAIRQSPSPPPEEESRGPGHPPLPDKFQFELTLEEEEEEEIS MAQIPCTAQEALTAQGLSGVEEALDATIAWEASPAQESLEVMAQEASLEA ELEAVYLTQQAQSTGSAPVAPDEFSSREEFVVAVSHSSPSALALQSPLLP AWRTLSVSEHAPGLPGLPSTAAEVEAQREHQAAKRACSACAGTFGEDT SALPAPGGGGSGGDPT |
| UniProtKB - P27815-2) | |
| SEQ ID NO:2 | |
| PDE4A (Human Isoform 3) | MCPFPVTTVPLGGPTPVCKATLSEETCQQLARETLEELDWCLEQLETMQ TYRSVSEMASHKFKRMLNRELTHLSEMSRSGNQVSEYISTTFLDKQNEV EIPSPTMKEREKQQAPRPRPSQPPPPPVPHLQPMSQITGLKKLMHSNSL NNSNIPRFGVKTDQEELLAQELENLNKWGLNIFCVSDYAGGRSLTCIMYM IFQERDLLKKFRIPVDTMVTYMLTLEDHYHADVAYHNSLHAADVLQSTHV LLATPALDAVFTDLEILAALFAAAIHDVDHPGVSNQFLINTNSELALMYNDE SVLENHHLAVGFKLLQEDNCDIFQNLSKRQRQSLRKMVIDMVLATDMSK HMTLLADLKTMVETKKVTSSGVLLLDNYSDRIQVLRNMVHCADLSNPTKP LELYRQWTDRIMAEFFQQGDRERERGMEISPMCDKHTASVEKSQVGFID YIVHPLWETWADLVHPDAQEILDTLEDNRDWYYSAIRQSPSPPPEEESR GPGHPPLPDKFQFELTLEEEEEEEISMAQIPCTAQEALTAQGLSGVEEAL DATIAWEASPAQESLEVMAQEASLEAELEAVYLTQQAQSTGSAPVAPDE FSSREEFVVAVSHSSPSALALQSPLLPAWRTLSVSEHAPGLPGLPSTAAE VEAQREHQAAKRACSACAGTFGEDTSALPAPGGGGSGGDPT |
| UniProtKB - P27815-3) | |
| SEQ ID NO:3 | |
| PDE4A (Human Isoform 4) | MPLVDFFCETCSKPWLVGWWDQFKRMLNRELTHLSEMSRSGNQVSEYI STTFLDKQNEVEIPSPTMKEREKQQAPRPRPSQPPPPPVPHLQPMSQIT GLKKLMHSNSLNNSNIPRFGVKTDQEELLAQELENLNKWGLNIFCVSDYA GGRSLTCIMYMIFQERDLLKKFRIPVDTMVTYMLTLEDHYHADVAYHNSL HAADVLQSTHVLLATPALDAVFTDLEILAALFAAAIHDVDHPGVSNQFLINT NSELALMYNDESVLENHHLAVGFKLLQEDNCDIFQNLSKRQRQSLRKMVI DMVLATDMSKHMTLLADLKTMVETKKVTSSGVLLLDNYSDRIQVLRNMV HCADLSNPTKPLELYRQWTDRIMAEFFQQGDRERERGMEISPMCDKHT ASVEKSQVGFIDYIVHPLWETWADLVHPDAQEILDTLEDNRDWYYSAIRQ SPSPPPEEESRGPGHPPLPDKFQFELTLEEEEEEEISMAQIPCTAQEALT AQGLSGVEEALDATIAWEASPAQESLEVMAQEASLEAELEAVYLTQQAQ STGSAPVAPDEFSSREEFVVAVSHSSPSALALQSPLLPAWRTLSVSEHAP GLPGLPSTAAEVEAQREHQAAKRACSACAGTFGEDTSALPAPGGGGSG GDPT |
| UniProtKB - P27815-4) | |
| SEQ ID NO:4 | |
| PDE4A (Human Isoform 5) | MVLPSDQGFKLLGNVLQGPEPYRLLTSGLRLHQELENLNKWGLNIFCVS DYAGGRSLTCIMYMIFQERDLLKKFRIPVDTMVTYMLTLEDHYHADVAYH NSLHAADVLQSTHVLLATPALDAVFTDLEILAALFAAAIHDVDHPGVSNQF |
| UniProtKB - P27815-5) | LINTNSELALMYNDESVLENHHLAVGFKLLQEDNCDIFQNLSKRQRQSLR KMVIDMVLATDMSKHMTLLADLKTMVETKKVTSSGVLLLDNYSDRIQVLR NMVHCADLSNPTKPLELYRQWTDRIMAEFFQQGDRERERGMEISPMCD KHTASVEKSQVQARGIDGRAQGGFY |
| SEQ ID NO:5 | |
| PDE4A (Human Isoform 6) | MRSGAAPRARPRPPALALPPTGPESLTHFPFSDEDTRRHPPGRSVSFEA ENGPTPSPGRSPLDSQASPGLVLHAGAATSQRRESFLYRSDSDYDMSP KTMSRNSSVTSEAHAEDLIVTPFAQVLASLRSVRSNFSLLTNVPVPSNKR SPLGGPTPVCKATLSEETCQQLARETLEELDWCLEQLETMQTYRSVSEM ASHKFKRMLNRELTHLSEMSRSGNQVSEYISTTFLDKQNEVEIPSPTMKE REKQQAPRPRPSQPPPPPVPHLQPMSQITGLKKLMHSNSLNNSNIPRFG VKTDQEELLAQELENLNKWGLNIFCVSDYAGGRSLTCIMYMIFQERDLLK KFRIPVDTMVTYMLTLEDHYHADVAYHNSLHAADVLQSTHVLLATPALDA VFTDLEILAALFAAAIHDVDHPGVSNQFLINTNSELALMYNDESVLENHHL AVGFKLLQEDNCDIFQNLSKRQRQSLRKMVIDMVLATDMSKHMTLLADLK TMVETKKVTSSGVLLLDNYSDRIQVLRNMVHCADLSNPTKPLELYRQWT DRIMAEFFQQGDRERERGMEISPMCDKHTASVEKSQVGFIDYIVHPLWE TWADLVHPDAQEILDTLEDNRDWYYSAIRQSPSPPPEEESRGPGHPPLP DKFQFELTLEEEEEEEISMAQIPCTAQEALTAQGLSGVEEALDATIAWEAS PAQESLEVMAQEASLEAELEAVYLTQQAQSTGSAPVAPDEFSSREEFVV AVSHSSPSALALQSPLLPAWRTLSVSEHAPGLPGLPSTAAEVEAQREHQ AAKRACSACAGTFGEDTSALPAPGGGGSGGDPT |
| UniProtKB - P27815-6) | |
| SEQ ID NO:6 | |
| PDE4A (Human Isoform 7) | MKRSRSALSVAGTGDERSRETPESDRANMLGADLRRPRRRLSSGPGLG WAQPEPSDPGVPLPPRPTTLPLLIPPRISITRAENDSFEAENGPTPSPGRS PLDSQASPGLVLHAGAATSQRRESFLYRSDSDYDMSPKTMSRNSSVTSE AHAEDLIVTPFAQVLASLRSVRSNFSLLTNVPVPSNKRSPLGGPTPVCKA TLSEETCQQLARETLEELDWCLEQLETMQTYRSVSEMASHKFKRMLNRE LTHLSEMSRSGNQVSEYISTTFLDKQNEVEIPSPTMKEREKQQAPRPRPS QPPPPPVPHLQPMSQITGLKKLMHSNSLNNSNIPRFGVKTDQEELLAQEL ENLNKWGLNIFCVSDYAGGRSLTCIMYMIFQERDLLKKFRIPVDTMVTYM LTLEDHYHADVAYHNSLHAADVLQSTHVLLATPALDAVFTDLEILAALFAA AIHDVDHPGVSNQFLINTNSELALMYNDESVLENHHLAVGFKLLQEDNCD IFQNLSKRQRQSLRKMVIDMVLATDMSKHMTLLADLKTMVETKKVTSSGV LLLDNYSDRIQVLRNMVHCADLSNPTKPLELYRQWTDRIMAEFFQQGDR ERERGMEISPMCDKHTASVEKSQVGFIDYIVHPLWETWADLVHPDAQEIL DTLEDNRDWYYSAIRQSPSPPPEEESRGPGHPPLPDKFQFELTLEEEEE EEISMAQIPCTAQEALTAQGLSGVEEALDATIAWEASPAQESLEVMAQEA SLEAELEAVYLTQQAQSTGSAPVAPDEFSSREEFVVAVSHSSPSALALQS PLLPAWRTLSVSEHAPGLPGLPSTAAEVEAQREHQAAKRACSACAGTFG EDTSALPAPGGGGSGGDPT |
| UniProtKB - P27815-7) | |
| SEQ ID NO:7 | |
| PDE4B (Human Isoform 1) | MKKSRSVMTVMADDNVKDYFECSLSKSYSSSSNTLGIDLWRGRRCCSG NLQLPPLSQRQSERARTPEGDGISRPTTLPLTTLPSIAITTVSQECFDVEN GPSPGRSPLDPQASSSAGLVLHATFPGHSQRRESFLYRSDSDYDLSPKA MSRNSSLPSEQHGDDLIVTPFAQVLASLRSVRNNFTILTNLHGTSNKRSP AASQPPVSRVNPQEESYQKLAMETLEELDWCLDQLETIQTYRSVSEMAS NKFKRMLNRELTHLSEMSRSGNQVSEYISNTFLDKQNDVEIPSPTQKDRE KKKKQQLMTQISGVKKLMHSSSLNNTSISRFGVNTENEDHLAKELEDLNK WGLNIFNVAGYSHNRPLTCIMYAIFQERDLLKTFRISSDTFITYMMTLEDH YHSDVAYHNSLHAADVAQSTHVLLSTPALDAVFTDLEILAAIFAAAIHDVD HPGVSNQFLINTNSELALMYNDESVLENHHLAVGFKLLQEEHCDIFMNLT KKQRQTLRKMVIDMVLATDMSKHMSLLADLKTMVETKKVTSSGVLLLDN YTDRIQVLRNMVHCADLSNPTKSLELYRQWTDRIMEEFFQQGDKERERG MEISPMCDKHTASVEKSQVGFIDYIVHPLWETWADLVQPDAQDILDTLED NRNWYQSMIPQSPSPPLDEQNRDCQGLMEKFQFELTLDEEDSEGPEKE GEGHSYFSSTKTLCVIDPENRDSLGETDIDIATEDKSPVDT |
| UniProtKB - Q07343-1) | |
| SEQ ID NO:8 | |
| PDE4B (Human Isoform 2) | MKEHGGTFSSTGISGGSGDSAMDSLQPLQPNYMPVCLFAEESYQKLAM ETLEELDWCLDQLETIQTYRSVSEMASNKFKRMLNRELTHLSEMSRSGN |
| UniProtKB - Q07343-2) | |
| SEQ ID NO:9 | |
| | QVSEYISNTFLDKQNDVEIPSPTQKDREKKKKQQLMTQISGVKKLMHSSS LNNTSISRFGVNTENEDHLAKELEDLNKWGLNIFNVAGYSHNRPLTCIMY AIFQERDLLKTFRISSDTFITYMMTLEDHYHSDVAYHNSLHAADVAQSTHV LLSTPALDAVFTDLEILAAIFAAAIHDVDHPGVSNQFLINTNSELALMYNDE SVLENHHLAVGFKLLQEEHCDIFMNLTKKQRQTLRKMVIDMVLATDMSKH MSLLADLKTMVETKKVTSSGVLLLDNYTDRIQVLRNMVHCADLSNPTKSL ELYRQWTDRIMEEFFQQGDKERERGMEISPMCDKHTASVEKSQVGFIDY IVHPLWETWADLVQPDAQDILDTLEDNRNWYQSMIPQSPSPPLDEQNRD CQGLMEKFQFELTLDEEDSEGPEKEGEGHSYFSSTKTLCVIDPENRDSL GETDIDIATEDKSPVDT |
| PDE4B (Human Isoform 3) | MTAKDSSKELTASEPEVCIKTFKEQMHLELELPRLPGNRPTSPKISPRSSP RNSPCFFRKLLVNKSIRQRRRFTVAHTCFDVENGPSPGRSPLDPQASSS AGLVLHATFPGHSQRRESFLYRSDSDYDLSPKAMSRNSSLPSEQHGDDL IVTPFAQVLASLRSVRNNFTILTNLHGTSNKRSPAASQPPVSRVNPQEES YQKLAMETLEELDWCLDQLETIQTYRSVSEMASNKFKRMLNRELTHLSE MSRSGNQVSEYISNTFLDKQNDVEIPSPTQKDREKKKKQQLMTQISGVK KLMHSSSLNNTSISRFGVNTENEDHLAKELEDLNKWGLNIFNVAGYSHNR PLTCIMYAIFQERDLLKTFRISSDTFITYMMTLEDHYHSDVAYHNSLHAAD VAQSTHVLLSTPALDAVFTDLEILAAIFAAAIHDVDHPGVSNQFLINTNSEL ALMYNDESVLENHHLAVGFKLLQEEHCDIFMNLTKKQRQTLRKMVIDMVL ATDMSKHMSLLADLKTMVETKKVTSSGVLLLDNYTDRIQVLRNMVHCADL SNPTKSLELYRQWTDRIMEEFFQQGDKERERGMEISPMCDKHTASVEKS QVGFIDYIVHPLWETWADLVQPDAQDILDTLEDNRNWYQSMIPQSPSPPL DEQNRDCQGLMEKFQFELTLDEEDSEGPEKEGEGHSYFSSTKTLCVIDP ENRDSLGETDIDIATEDKSPVDT |
| UniProtKB - Q07343-3) | |
| SEQ ID NO:10 | |
| PDE4B (Human Isoform 5) | MPEANYLLSVSWGYIKFKRMLNRELTHLSEMSRSGNQVSEYISNTFLDK QNDVEIPSPTQKDREKKKKQQLMTQISGVKKLMHSSSLNNTSISRFGVNT ENEDHLAKELEDLNKWGLNIFNVAGYSHNRPLTCIMYAIFQERDLLKTFRI SSDTFITYMMTLEDHYHSDVAYHNSLHAADVAQSTHVLLSTPALDAVFTD LEILAAIFAAAIHDVDHPGVSNQFLINTNSELALMYNDESVLENHHLAVGFK LLQEEHCDIFMNLTKKQRQTLRKMVIDMVLATDMSKHMSLLADLKTMVET KKVTSSGVLLLDNYTDRIQVLRNMVHCADLSNPTKSLELYRQWTDRIMEE FFQQGDKERERGMEISPMCDKHTASVEKSQVGFIDYIVHPLWETWADLV QPDAQDILDTLEDNRNWYQSMIPQSPSPPLDEQNRDCQGLMEKFQFELT LDEEDSEGPEKEGEGHSYFSSTKTLCVIDPENRDSLGETDIDIATEDKSPV DT |
| UniProtKB - Q07343-4) | |
| SEQ ID NO:11 | |
| PDE4C (Human Isoform 1) | MENLGVGEGAEACSRLSRSRGRHSMTRAPKHLWRQPRRPIRIQQRFYS DPDKSAGCRERDLSPRPELRKSRLSWPVSSCRRFDLENGLSCGRRALD PQSSPGLGRIMQAPVPHSQRRESFLYRSDSDYELSPKAMSRNSSVASDL HGEDMIVTPFAQVLASLRTVRSNVAALARQQCLGAAKQGPVGNPSSSNQ LPPAEDTGQKLALETLDELDWCLDQLETLQTRHSVGEMASNKFKRILNRE LTHLSETSRSGNQVSEYISRTFLDQQTEVELPKVTAEEAPQPMSRISGLH GLCHSASLSSATVPRFGVQTDQEEQLAKELEDTNKWGLDVFKVAELSGN RPLTAIIFSIFQERDLLKTFQIPADTLATYLLMLEGHYHANVAYHNSLHAAD VAQSTHVLLATPALEAVFTDLEILAALFASAIHDVDHPGVSNQFLINTNSEL ALMYNDASVLENHHLAVGFKLLQAENCDIFQNLSAKQRLSLRRMVIDMVL ATDMSKHMNLLADLKTMVETKKVTSLGVLLLDNYSDRIQVLQNLVHCADL SNPTKPLPLYRQWTDRIMAEFFQQGDRERESGLDISPMCDKHTASVEKS QVGFIDYIAHPLWETWADLVHPDAQDLLDTLEDNREWYQSKIPRSPSDLT NPERDGPDRFQFELTLEEAEEEDEEEEEEGEETALAKEALELPDTELLSP EAGPDPGDLPLDNQRT |
| UniProtKB - Q08493-1) | |
| SEQ ID NO:12 | |
| PDE4C (Human Isoform 2) | MQAPVPHSQRRESFLYRSDSDYELSPKAMSRNSSVASDLHGEDMIVTPF AQVLASLRTVRSNVAALARQQCLGAAKQGPVGNPSSSNQLPPAEDTGQ KLALETLDELDWCLDQLETLQTRHSVGEMASNKFKRILNRELTHLSETSR SGNQVSEYISRTFLDQQTEVELPKVTAEEAPQPMSRISGLHGLCHSASLS SATVPRFGVQTDQEEQLAKELEDTNKWGLDVFKVAELSGNRPLTAIIFSIF |
| UniProtKB - Q08493-2) | |
| SEQ ID NO:13 | |
| | QERDLLKTFQIPADTLATYLLMLEGHYHANVAYHNSLHAADVAQSTHVLL ATPALEAVFTDLEILAALFASAIHDVDHPGVSNQFLINTNSELALMYNDASV LENHHLAVGFKLLQAENCDIFQNLSAKQRLSLRRMVIDMVLATDMSKHM NLLADLKTMVETKKVTSLGVLLLDNYSDRIQVLQNLVHCADLSNPTKPLPL YRQWTDRIMAEFFQQGDRERESGLDISPMCDKHTASVEKSQVGFIDYIA HPLWETWADLVHPDAQDLLDTLEDNREWYQSKIPRSPSDLTNPERDGP DRFQFELTLEEAEEEDEEEEEEGEETALAKEALELPDTELLSPEAGPDPG DLPLDNQRT |
| PDE4C (Human Isoform 3) | MQGPPAPAPVPGPGSPRGSPRGSPGLFRKLLVNQSIRLQRRFTVAHPLC FDLENGLSCGRRALDPQSSPGLGRIMQAPVPHSQRRESFLYRSDSDYEL SPKAMSRNSSVASDLHGEDMIVTPFAQVLASLRTVRSNVAALARQQCLG AAKQGPVGNPSSSNQLPPAEDTGQKLALETLDELDWCLDQLETLQTRHS VGEMASNKFKRILNRELTHLSETSRSGNQVSEYISRTFLDQQTEVELPKV TAEEAPQPMSRISGLHGLCHSASLSSATVPRFGVQTDQEEQLAKELEDT NKWGLDVFKVAELSGNRPLTAIIFSIFQERDLLKTFQIPADTLATYLLMEG HYHANVAYHNSLHAADVAQSTHVLLATPALEAVFTDLEILAALFASAIHDV DHPGVSNQFLINTNSELALMYNDASVLENHHLAVGFKLLQAENCDIFQNL SAKQRLSLRRMVIDMVLATDMSKHMNLLADLKTMVETKKVTSLGVLLLDN YSDRIQVLQNLVHCADLSNPTKPLPLYRQWTDRIMAEFFQQGDRERESG LDISPMCDKHTASVEKSQVGFIDYIAHPLWETWADLVHPDAQDLLDTLED NREWYQSKIPRSPSDLTNPERDGPDRFQFELTLEEAEEEDEEEEEEGEE TALAKEALELPDTELLSPEAGPDPGDLPLDNQRT |
| UniProtKB - Q08493-3) | |
| SEQ ID NO:14 | |
| PDE4D (Human Isoform 4) | |
| UniProtKB - Q08499-1) | |
| SEQ ID NO:15 | |
| PDE4D (Human Isoform 3) | MMHVNNFPFRRHSWICFDVDNGTSAGRSPLDPMTSPGSGLILQANFVHS QRRESFLYRSDSDYDLSPKSMSRNSSIASDIHGDDLIVTPFAQVLASLRTV RNNFAALTNLQDRAPSKRSPMCNQPSINKATITEEAYQKLASETLEELDW CLDQLETLQTRHSVSEMASNKFKRMLNRELTHLSEMSRSGNQVSEFISN TFLDKQHEVEIPSPTQKEKEKKKRPMSQISGVKKLMHSSSLTNSSIPRFG VKTEQEDVLAKELEDVNKWGLHVFRIAELSGNRPLTVIMHTIFQERDLLKT FKIPVDTLITYLMTLEDHYHADVAYHNNIHAADVVQSTHVLLSTPALEAVFT DLEILAAIFASAIHDVDHPGVSNQFLINTNSELALMYNDSSVLENHHLAVGF KLLQEENCDIFQNLTKKQRQSLRKMVIDIVLATDMSKHMNLLADLKTMVE TKKVTSSGVLLLDNYSDRIQVLQNMVHCADLSNPTKPLQLYRQWTDRIM EEFFRQGDRERERGMEISPMCDKHNASVEKSQVGFIDYIVHPLWETWAD LVHPDAQDILDTLEDNREWYQSTIPQSPSPAPDDPEEGRQGQTEKFQFE LTLEEDGESDTEKDSGSQVEEDTSCSDSKTLCTQDSESTEIPLDEQVEEE AVGEEEESQPEACVIDDRSPDT |
| UniProtKB - Q08499-2) | |
| SEQ ID NO:16 | |
| PDE4D (Human Isoform 10) | MSRNSSIASDIHGDDLIVTPFAQVLASLRTVRNNFAALTNLQDRAPSKRSP MCNQPSINKATITEEAYQKLASETLEELDWCLDQLETLQTRHSVSEMASN KFKRMLNRELTHLSEMSRSGNQVSEFISNTFLDKQHEVEIPSPTQKEKEK |
| UniProtKB - Q08499-3) | |
| SEQ ID NO:17 | |
| | KKRPMSQISGVKKLMHSSSLTNSSIPRFGVKTEQEDVLAKELEDVNKWG LHVFRIAELSGNRPLTVIMHTIFQERDLLKTFKIPVDTLITYLMTLEDHYHAD VAYHNNIHAADVVQSTHVLLSTPALEAVFTDLEILAAIFASAIHDVDHPGVS NQFLINTNSELALMYNDSSVLENHHLAVGFKLLQEENCDIFQNLTKKQRQ SLRKMVIDIVLATDMSKHMNLLADLKTMVETKKVTSSGVLLLDNYSDRIQV LQNMVHCADLSNPTKPLQLYRQWTDRIMEEFFRQGDRERERGMEISPM CDKHNASVEKSQVGFIDYIVHPLWETWADLVHPDAQDILDTLEDNREWY QSTIPQSPSPAPDDPEEGRQGQTEKFQFELTLEEDGESDTEKDSGSQVE EDTSCSDSKTLCTQDSESTEIPLDEQVEEEAVGEEEESQPEACVIDDRSP DT |
| PDE4D (Human Isoform 1) | MKEQPSCAGTGHPMAGYGRMAPFELASGPVKRLRTESPFPCLFAEEAY QKLASETLEELDWCLDQLETLQTRHSVSEMASNKFKRMLNRELTHLSEM SRSGNQVSEFISNTFLDKQHEVEIPSPTQKEKEKKKRPMSQISGVKKLMH SSSLTNSSIPRFGVKTEQEDVLAKELEDVNKWGLHVFRIAELSGNRPLTVI MHTIFQERDLLKTFKIPVDTLITYLMTLEDHYHADVAYHNNIHAADVVQST HVLLSTPALEAVFTDLEILAAIFASAIHDVDHPGVSNQFLINTNSELALMYN DSSVLENHHLAVGFKLLQEENCDIFQNLTKKQRQSLRKMVIDIVLATDMS KHMNLLADLKTMVETKKVTSSGVLLLDNYSDRIQVLQNMVHCADLSNPTK PLQLYRQWTDRIMEEFFRQGDRERERGMEISPMCDKHNASVEKSQVGFI DYIVHPLWETWADLVHPDAQDILDTLEDNREWYQSTIPQSPSPAPDDPE EGRQGQTEKFQFELTLEEDGESDTEKDSGSQVEEDTSCSDSKTLCTQD SESTEIPLDEQVEEEAVGEEEESQPEACVIDDRSPDT |
| UniProtKB - Q08499-4) | |
| SEQ ID NO:18 | |
| PDE4D (Human Isoform 2) | MASNKFKRMLNRELTHLSEMSRSGNQVSEFISNTFLDKQHEVEIPSPTQK EKEKKKRPMSQISGVKKLMHSSSLTNSSIPRFGVKTEQEDVLAKELEDVN KWGLHVFRIAELSGNRPLTVIMHTIFQERDLLKTFKIPVDTLITYLMTLEDH YHADVAYHNNIHAADVVQSTHVLLSTPALEAVFTDLEILAAIFASAIHDVDH PGVSNQFLINTNSELALMYNDSSVLENHHLAVGFKLLQEENCDIFQNLTK KQRQSLRKMVIDIVLATDMSKHMNLLADLKTMVETKKVTSSGVLLLDNYS DRIQVLQNMVHCADLSNPTKPLQLYRQWTDRIMEEFFRQGDRERERGM EISPMCDKHNASVEKSQVGFIDYIVHPLWETWADLVHPDAQDILDTLEDN REWYQSTIPQSPSPAPDDPEEGRQGQTEKFQFELTLEEDGESDTEKDS GSQVEEDTSCSDSKTLCTQDSESTEIPLDEQVEEEAVGEEEESQPEACVI DDRSPDT |
| UniProtKB - Q08499-5) | |
| SEQ ID NO:19 | |
| PDE4D (Human Isoform 5) | MAQQTSPDTLTVPEVDNPHCPNPWLNEDLVKSLRENLLQHEKSKTARKS VSPKLSPVISPRNSPRLLRRMLLSSNIPKQRRFTVAHTCFDVDNGTSAGR SPLDPMTSPGSGLILQANFVHSQRRESFLYRSDSDYDLSPKSMSRNSSIA SDIHGDDLIVTPFAQVLASLRTVRNNFAALTNLQDRAPSKRSPMCNQPSI NKATITEEAYQKLASETLEELDWCLDQLETLQTRHSVSEMASNKFKRMLN RELTHLSEMSRSGNQVSEFISNTFLDKQHEVEIPSPTQKEKEKKKRPMSQ ISGVKKLMHSSSLTNSSIPRFGVKTEQEDVLAKELEDVNKWGLHVFRIAEL SGNRPLTVIMHTIFQERDLLKTFKIPVDTLITYLMTLEDHYHADVAYHNNIH AADVVQSTHVLLSTPALEAVFTDLEILAAIFASAIHDVDHPGVSNQFLINTN SELALMYNDSSVLENHHLAVGFKLLQEENCDIFQNLTKKQRQSLRKMVIDI VLATDMSKHMNLLADLKTMVETKKVTSSGVLLLDNYSDRIQVLQNMVHC ADLSNPTKPLQLYRQWTDRIMEEFFRQGDRERERGMEISPMCDKHNAS VEKSQVGFIDYIVHPLWETWADLVHPDAQDILDTLEDNREWYQSTIPQSP SPAPDDPEEGRQGQTEKFQFELTLEEDGESDTEKDSGSQVEEDTSCSD SKTLCTQDSESTEIPLDEQVEEEAVGEEEESQPEACVIDDRSPDT |
| UniProtKB - Q08499-6) | |
| SEQ ID NO:20 | |
| PDE4D (Human Isoform N3) | MAQQTSPDTLTVPEVDNPHCPNPWLNEDLVKSLRENLLQHEKSKTARKS VSPKLSPVISPRNSPRLLRRMLLSSNIPKQRRFTVAHTCFDVDNGTSAGR SPLDPMTSPGSGLILQANFVHSQRRESFLYRSDSDYDLSPKSMSRNSSIA SDIHGDDLIVTPFAQVLASLRTVRNNFAALTNLQDRAPSKRSPMCNQPSI NKATITGLYNGIIAFL |
| UniProtKB - Q08499-7) | |
| SEQ ID NO:21 | |
| PDE4D (Human Isoform 6) | MPEANYLLSVSWGYIKFKRMLNRELTHLSEMSRSGNQVSEFISNTFLDKQ HEVEIPSPTQKEKEKKKRPMSQISGVKKLMHSSSLTNSSIPRFGVKTEQE |
| UniProtKB - Q08499-8) | |
| SEQ ID NO:22 | |
| | DVLAKELEDVNKWGLHVFRIAELSGNRPLTVIMHTIFQERDLLKTFKIPVDT LITYLMTLEDHYHADVAYHNNIHAADVVQSTHVLLSTPALEAVFTDLEILAA IFASAIHDVDHPGVSNQFLINTNSELALMYNDSSVLENHHLAVGFKLLQEE NCDIFQNLTKKQRQSLRKMVIDIVLATDMSKHMNLLADLKTMVETKKVTS SGVLLLDNYSDRIQVLQNMVHCADLSNPTKPLQLYRQWTDRIMEEFFRQ GDRERERGMEISPMCDKHNASVEKSQVGFIDYIVHPLWETWADLVHPDA QDILDTLEDNREWYQSTIPQSPSPAPDDPEEGRQGQTEKFQFELTLEED GESDTEKDSGSQVEEDTSCSDSKTLCTQDSESTEIPLDEQVEEEAVGEE EESQPEACVIDDRSPDT |
| PDE4D (Human Isoform 8) | MAFVWDPLGATVPGPSTRAKSRLRFSKSYSFDVDNGTSAGRSPLDPMT SPGSGLILQANFVHSQRRESFLYRSDSDYDLSPKSMSRNSSIASDIHGDD LIVTPFAQVLASLRTVRNNFAALTNLQDRAPSKRSPMCNQPSINKATITEE AYQKLASETLEELDWCLDQLETLQTRHSVSEMASNKFKRMLNRELTHLS EMSRSGNQVSEFISNTFLDKQHEVEIPSPTQKEKEKKKRPMSQISGVKKL MHSSSLTNSSIPRFGVKTEQEDVLAKELEDVNKWGLHVFRIAELSGNRPL TVIMHTIFQERDLLKTFKIPVDTLITYLMTLEDHYHADVAYHNNIHAADVVQ STHVLLSTPALEAVFTDLEILAAIFASAIHDVDHPGVSNQFLINTNSELALM YNDSSVLENHHLAVGFKLLQEENCDIFQNLTKKQRQSLRKMVIDIVLATD MSKHMNLLADLKTMVETKKVTSSGVLLLDNYSDRIQVLQNMVHCADLSN PTKPLQLYRQWTDRIMEEFFRQGDRERERGMEISPMCDKHNASVEKSQ VGFIDYIVHPLWETWADLVHPDAQDILDTLEDNREWYQSTIPQSPSPAPD DPEEGRQGQTEKFQFELTLEEDGESDTEKDSGSQVEEDTSCSDSKTLCT QDSESTEIPLDEQVEEEAVGEEEESQPEACVIDDRSPDT |
| UniProtKB - Q08499-9) | |
| SEQ ID NO:23 | |
| PDE4D (Human Isoform 9) | MSIIMKPRSRSTSSLRTAEAVCEDVDNGTSAGRSPLDPMTSPGSGLILQA NFVHSQRRESFLYRSDSDYDLSPKSMSRNSSIASDIHGDDLIVTPFAQVL ASLRTVRNNFAALTNLQDRAPSKRSPMCNQPSINKATITEEAYQKLASET LEELDWCLDQLETLQTRHSVSEMASNKFKRMLNRELTHLSEMSRSGNQ VSEFISNTFLDKQHEVEIPSPTQKEKEKKKRPMSQISGVKKLMHSSSLTNS SIPRFGVKTEQEDVLAKELEDVNKWGLHVFRIAELSGNRPLTVIMHTIFQE RDLLKTFKIPVDTLITYLMTLEDHYHADVAYHNNIHAADVVQSTHVLLSTPA LEAVFTDLEILAAIFASAIHDVDHPGVSNQFLINTNSELALMYNDSSVLENH HLAVGFKLLQEENCDIFQNLTKKQRQSLRKMVIDIVLATDMSKHMNLLADL KTMVETKKVTSSGVLLLDNYSDRIQVLQNMVHCADLSNPTKPLQLYRQW TDRIMEEFFRQGDRERERGMEISPMCDKHNASVEKSQVGFIDYIVHPLW ETWADLVHPDAQDILDTLEDNREWYQSTIPQSPSPAPDDPEEGRQGQTE KFQFELTLEEDGESDTEKDSGSQVEEDTSCSDSKTLCTQDSESTEIPLDE QVEEEAVGEEEESQPEACVIDDRSPDT |
| UniProtKB - Q08499-10) | |
| SEQ ID NO:24 | |
| PDE4D (Human Isoform 7) | MKRNTCDLLSRSKSASEETLHSSNEEEDPFRGMEPYLVRRLSCRNIQLP PLAFRQLEQADLKSESENIQRPTSLPLKILPLIAITSAESSGFDVDNGTSAG RSPLDPMTSPGSGLILQANFVHSQRRESFLYRSDSDYDLSPKSMSRNSSI ASDIHGDDLIVTPFAQVLASLRTVRNNFAALTNLQDRAPSKRSPMCNQPS INKATITEEAYQKLASETLEELDWCLDQLETLQTRHSVSEMASNKFKRML NRELTHLSEMSRSGNQVSEFISNTFLDKQHEVEIPSPTQKEKEKKKRPMS QISGVKKLMHSSSLTNSSIPRFGVKTEQEDVLAKELEDVNKWGLHVFRIA ELSGNRPLTVIMHTIFQERDLLKTFKIPVDTLITYLMTLEDHYHADVAYHNN IHAADVVQSTHVLLSTPALEAVFTDLEILAAIFASAIHDVDHPGVSNQFLINT NSELALMYNDSSVLENHHLAVGFKLLQEENCDIFQNLTKKQRQSLRKMVI DIVLATDMSKHMNLLADLKTMVETKKVTSSGVLLLDNYSDRIQVLQNMVH CADLSNPTKPLQLYRQWTDRIMEEFFRQGDRERERGMEISPMCDKHNA SVEKSQVGFIDYIVHPLWETWADLVHPDAQDILDTLEDNREWYQSTIPQS PSPAPDDPEEGRQGQTEKFQFELTLEEDGESDTEKDSGSQVEEDTSCS DSKTLCTQDSESTEIPLDEQVEEEAVGEEEESQPEACVIDDRSPDT |
| UniProtKB - Q08499-11) | |
| SEQ ID NO:25 | |
| PDE4D (Human Isoform 12) | MAQQTSPDTLTVPEVDNPHCPNPWLNEDLVKSLRENLLQHEKSKTARKS VSPKLSPVISPRNSPRLLRRMLLSSNIPKQRRFTVAHTCFDVDNGTSAGR SPLDPMTSPGSGLILQANFVHSQRRESFLYRSDSDYDLSPKSMSRNSSIA |
| UniProtKB - Q08499-12) | |
| SEQ ID NO:26 | |
| | SDIHGDDLIVTPFAQVLASLRTVRNNFAALTNLQDRAPSKRSPMCNQPSI NKATITGSWMELNPYTLLDM |

As used herein, a "PDE4 inhibitor" or an "inhibitor of PDE4" is an agent that inhibits (e.g., reduces or abolishes) phosphodiesterase activity of one or more PDE4 isoforms as compared to the phosphodiesterase activity of the one or more PDE4 isoforms in the absence of the agent. In certain embodiments, the inhibitor results in the phosphodiesterase activity of at least one of the one or more PDE4 isoforms being 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, 5% or less, 4% or less, 3% or less, 2% or less, or 1% or less, as compared to the phosphodiesterase activity of the at least one of the one or more PDE4 isoforms in the absence of the inhibitor.

PDE4A is expressed in the CNS, including in the cortex, hippocampus, and cerebellum. PDE4B is widely expressed in the CNS, including in the striatum, amygdala, thalamus, and hypothalamus. PDE4C is not widely expressed in the CNS, although expression has been observed in the olfactory bulb with limited expression in the cortex. PDE4D is expressed in the CNS, including in the hippocampus and elsewhere.

In some instances, the methods include administering a PDE4 inhibitor that inhibits one or more of PDE4A, PDE4B, PDE4C and PDE4D. In certain instances, the PDE4 inhibitor inhibits two, three, or each of PDE4A, PDE4B, PDE4C and PDE4D. The methods may include administering a PDE4 inhibitor that exhibits selectivity among PDE4A, PDE4B, PDE4C, and PDE4D. By "selectivity" is meant the PDE4 inhibitor either: exclusively inhibits PDE4A, PDE4B, PDE4C, or PDE4D; or inhibits two or more of PDE4A, PDE4B, PDE4C, and PDE4D, where the inhibition of at least one of the two or more PDE4 isoforms is greater than the inhibition of a different isoform among the two or more PDE4 isoforms inhibited by the inhibitor. In some embodiments, the PDE4 inhibitor is selective for a PDE4 isoform. As used herein, a PDE4 inhibitor is "selective for" a PDE4 isoform when the inhibitor either: exclusively inhibits the PDE4 isoform (PDE4A, PDE4B, PDE4C, or PDE4D); or inhibits the PDE4 isoform to a greater extent than at least one other PDE4 isoform. For example, in certain embodiments, the PDE4 inhibitor is selective for PDE4B such that the inhibition of PDE4B is greater than the inhibition of a second PDE4 isoform, e.g., greater than the inhibition of PDE4D.

In some embodiments, the PDE4 inhibitor does not inhibit (or does not substantially inhibit) one, two, or three of PDE4A, PDE4C, and PDE4D. As used herein, an inhibitor does not "substantially inhibit" the activity of a PDE4 isoform when contacting the PDE4 isoform with the inhibitor does not inhibit the activity of the isoform by more than 20%, 15%, 10%, 5%, 4%, 3%, 2%, or 1%, as compared to the activity of the isoform in the absence of the inhibitor. In certain embodiments, the methods include administering a PDE4B inhibitor that does not inhibit (or does not substantially inhibit) PDE4D.

In some embodiments, the PDE4 inhibitor acts by binding to the catalytic domain (e.g., the active site) of PDE4. In other embodiments, the PDE4 inhibitor acts by allosterically inhibiting PDE4B activity. All eleven PDE superfamily members (PDE1-11) exhibit a high degree of sequence conservation across the catalytic domain, making PDE families distinguished instead by motifs that encode unique regulatory domains. Traditional strategies to target PDEs have largely focused on ligands that bind the catalytic domain, thereby leading to complete inhibition of cAMP hydrolysis and associated toxicities, as well as a narrow therapeutic range due to nonselective binding. PDE4A, PDE4B, PDE4C, and PDE4D each contain three signature regulatory domains: upstream conserved regions 1 (UCR1) and 2 (UCR2) forming a regulatory module, and a control region (CR3) domain at the C-terminus. PDE4B and PDE4D crystal structures show that phosphodiesterase activity is regulated by UCR2 allosteric control of the catalytic active site. Allosteric modulation of PDE4s is therefore an underappreciated approach to selectively interfere with PDE4 activity. Indeed, small molecule PDE4D allosteric modulators are potent in cellular and *in vivo* assays. Further, the UCR2 of PDE4B has been shown to intimately interact in *trans* with the active site in the catalytic domain. According to the invention, the methods include administering an inhibitor that selectively inhibits PDE4B (e.g., versus PDE4D), e.g., by binding to PDE4B in a manner in which the UCR2 closes over the active site, thereby preventing access by cAMP.

In some embodiments, the PDE4 inhibitor is an allosteric modulator that selectively inhibits PDE4B (e.g., versus PDE4D) by capture of the C-terminal regulatory helix (CR3) across the active site in a conformation that closes access by cAMP. For example, small molecules can interact with different residues along the CR3 helix resulting in multiple "closed" conformations. Fox et al. (2014) Cell Signal. 26(3):657-63. The CR3 helix can adopt slightly different orientations across the active site, each with unique helical registries. Examples of allosteric inhibitors that exhibit selectivity for PDE4B versus PDE4D and may be administered according to the methods of the present disclosure are known and include the 2-arylpyrimidine derivative compound A-33 (as described in Hagan et al. (2014) Bioorg Med Chem Lett. 24(16):4031-4 and Fox et al. (2014) Cell Signal. 26(3):657-63)) and the triazine compounds described in Hagan et al. (2014) Bioorg Med Chem Lett. 24(16):4031-4.

Any suitable type of PDE4 inhibitor may be employed when practicing the methods of the present disclosure. In some embodiments, the PDE4 inhibitor is a small molecule. By "small molecule" is meant a compound having a molecular weight of 1000 atomic mass units (amu) or less. In some embodiments, the small molecule is 750 amu or less, 500 amu or less, 400 amu or less, 300 amu or less, or 200 amu or less. In certain embodiments, the small molecule is not made of repeating molecular units such as are present in a polymer. Small molecule allosteric modulators of PDE4 activity which may be administered according to the methods of the present disclosure are known and include those described in Gurney et al. (2011) Handb Exp Pharmacol. 204:167-92; Burgin et al. (2010) Nat Biotechnol. 28(1):63-70; Fox et al. (*supra*); Hagan et al. (*supra*); and elsewhere. In some embodiments, when the PDE4 inhibitor is a small molecule, the PDE4 inhibitor is selected from AN2728 (5-(4-cyanophenoxy)-2,3-dihydro-1-hydroxy-2,1-benzoxaborole), drotaverine, ibudilast, irsogladine, piclamilast, roflumilast, theophylline, apremilast, compound A-33 (as described in Hagan et al. (2014) Bioorg Med Chem Lett. 24(16):4031-4 and Fox et al. (2014) Cell Signal. 26(3):657-63), a triazine compound as described in Hagan et al. (2014) Bioorg Med Chem Lett. 24(16):4031-4, and any combination thereof. In certain embodiments, when the PDE4 inhibitor is a small molecule, the PDE4 inhibitor is AN2728. The PDE4 inhibitor is not rolipram. The PDE4 inhibitor is not rolipram and exhibits a selectivity among PDE4A, PDE4B, PDE4C and PDE4D which is different from the selectivity of rolipram. In some embodiments, the methods employ a PDE4 inhibitor that exhibits less inhibition of PDE4D than rolipram.

According to some embodiments, the PDE4 inhibitor inhibits expression of PDE4. For example, the PDE4 inhibitor may inhibit expression of one, two, three, or each of PDE4A, PDE4B, PDE4C, and PDE4D. In certain embodiments, when the PDE4 inhibitor inhibits expression of PDE4, the PDE4 inhibitor is a nucleic acid-based inhibitor. By "nucleic acid-based inhibitor" is meant a polymer of two or more linked nucleotides, where the polymer may include naturally occurring nucleotides, non-naturally occurring nucleotides (e.g., nucleotide analogs such as LNA, FANA, 2'-O-Me RNA, 2'-fluoro RNA, and/or the like), or a combination thereof.

In some embodiments, a nucleic acid-based PDE4 inhibitor includes a region complementary to a portion of a messenger RNA (mRNA) that encodes PDE4A, a mRNA that encodes PDE4B, a mRNA that encodes PDE4C, a mRNA that encodes PDE4D, or any combination thereof. The term "complementary" as used herein refers to a nucleotide sequence that base-pairs by non-covalent bonds to all or a region of a target nucleic acid. In the canonical Watson-Crick base pairing, adenine (A) forms a base pair with thymine (T), as does guanine (G) with cytosine (C) in DNA. In RNA, thymine is replaced by uracil (U). As such, A is complementary to T and G is complementary to C. In RNA, A is complementary to U and vice versa. Typically, "complementary" refers to a nucleotide sequence that is at least partially complementary. The term "complementary" may also encompass duplexes that are fully complementary such that every nucleotide in one strand is complementary to every nucleotide in the other strand in corresponding positions. In certain cases, a nucleotide sequence may be partially complementary to a target, in which not all nucleotides are complementary to every nucleotide in the target nucleic acid in all the corresponding positions. For example, a primer may be perfectly (i.e., 100%) complementary to the target nucleic acid, or the primer and the target nucleic acid may share some degree of complementarity which is less than perfect (e.g., 70%, 75%, 85%, 90%, 95%, 99%). The percent identity of two nucleotide sequences can be determined by aligning the sequences for optimal comparison purposes (e.g., gaps can be introduced in the sequence of a first sequence for optimal alignment). The nucleotides at corresponding positions are then compared, and the percent identity between the two sequences is a function of the number of identical positions shared by the sequences (i.e., % identity= # of identical positions/total # of positions×100). When a position in one sequence is occupied by the same nucleotide as the corresponding position in the other sequence, then the molecules are identical at that position. A non-limiting example of such a mathematical algorithm is described in Karlin et al., Proc. Natl. Acad. Sci. USA 90:5873-5877 (1993). Such an algorithm is incorporated into the NBLAST and XBLAST programs (version 2.0) as described in Altschul et al., Nucleic Acids Res. 25:389-3402 (1997). When utilizing BLAST and Gapped BLAST programs, the default parameters of the respective programs (e.g., NBLAST) can be used. In one aspect, parameters for sequence comparison can be set at score=100, wordlength=12, or can be varied (e.g., wordlength=5 or wordlength=20).

According to some embodiments, the nucleotide sequence of a nucleic acid-based PDE4 inhibitor is selected/designed such that the nucleic acid-based PDE4 inhibitor is selective for a PDE4 isoform. For example, the nucleotide sequence of a nucleic acid-based PDE4 inhibitor may be selected/designed such that the inhibitor selectively inhibits expression of PDE4A, PDE4B, and/or PDE4C (e.g., versus PDE4D). In some embodiments, the nucleotide sequence is selected/designed such that the inhibitor selectively inhibits PDE4B expression, e.g., selectively inhibits PDE4B expression versus PDE4D expression.

Non-limiting examples of nucleic acid-based inhibitors that may be employed when practicing the methods of the present disclosure include short interfering RNAs (siRNA), microRNAs (miRNA), morpholinos, and/or the like. Based on the available sequence information for PDE4A (NCBI Gene ID: 5141 for human PDE4A), PDE4B (NCBI Gene ID: 5142 for human PDE4B), PDE4C (NCBI Gene ID: 5143 for human PDE4C) and PDE4D (NCBI Gene ID: 5144 for human PDE4C), and the corresponding transcripts, nucleic acid-based inhibitors such as siRNAs, miRNAs, morpholinos, etc. may be designed using available tools, e.g., siRNA Wizard from Invivogen, siDESIGN Center from Dharmacon, BLOCK-iT^{™} RNAi Designer from Invitrogen, miR-Synth available at microrna.osumc.edu/mir-synth, WMD3 - Web MicroRNA Designer, a morpholino design tool provided by Gene Tools, etc. Approaches for designing and delivering siRNAs, miRNAs, morpholinos, etc. for targeting a particular mRNA are known and described, e.g., in Chakraborty et al. (2017) Mol Ther Nucleic Acids 8:132-143; Ahmadzada et al. (2018) Biophys Rev. 10(1):69-86; Zheng et al. (2018) Trends Biotechnol. 36(5):562-575; Mohanty et al. (2015) Curr Pharm Des. 21(31):4606-13; Gomes et al. (2015) Ageing Res Rev. 21:43-54; Gustincich et al. (2017) Prog Neurobiol. 155:194-211; Monsoori et al. (2014) Adv Pharm Bull. 4(4):313-321; and Xin et al. (2017) Mol Cancer 16:134.

As summarized above, aspects of the present disclosure relate to a phosphodiesterase 4 (PDE4) inhibitor for use in a method of treating epilepsy (sometimes referred to as "seizure disorder") by administering to an individual having epilepsy (e.g., an individual diagnosed as having epilepsy) a therapeutically effective amount of a PDE4 inhibitor. In some embodiments, the PDE4 inhibitor is any PDE4 inhibitor described elsewhere herein, including any PDE4 inhibitor identified using the methods of identifying anti-epileptic agents of the present disclosure. The older established anti-epileptic drugs (AEDs) phenytoin, carbamazepine, clonazepam, ethosuximide, valproic acid and barbiturates are widely prescribed but suffer from a range of side effects. In addition, there is a significant group of patients (30-40%) that are resistant to the currently available therapeutic agents. Several more recent drugs have been launched, including felbamate, gabapentin, lamotrigine, oxcarbazepine, tiagabine, topiramate, vigabartrin, zonisamide and levetiracetam. While some such more recent drugs show improved efficacies and side-effect profiles, about 30% of patients with epilepsy remain untreated.

In view of the unexpected findings described herein that PDE4 inhibition blocks a bioenergetics and hyperexcitable neuronal phenotype in both zebrafish and mouse models of epilepsy, protects against hyperthermia-induced seizures in a mouse model of epilepsy, and blocks seizures using a 6Hz test, it will be appreciated that the methods find use in treating a wide variety of epilepsies. In certain embodiments, the individual has an epilepsy selected from benign Rolandic epilepsy, frontal lobe epilepsy, infantile spasms, juvenile myoclonic epilepsy (JME), juvenile absence epilepsy, childhood absence epilepsy, pyknolepsy, febrile seizures, progressive myoclonus epilepsy of Lafora, Lennox-Gastaut syndrome, Landau-Kleffner syndrome, Dravet syndrome (DS), Generalized Epilepsy with Febrile Seizures (GEFS+), Severe Myoclonic Epilepsy of Infancy (SMEI), Benign Neonatal Familial Convulsions (BFNC), West Syndrome, Ohtahara Syndrome, early myoclonic encephalopathy, migrating partial epilepsy, infantile epileptic encephalopathies, Tuberous Sclerosis Complex (TSC), focal cortical dysplasia, Type I Lissencephaly, Miller-Dieker Syndrome, Angelman's syndrome, Fragile X syndrome, epilepsy in autism spectrum disorders, subcortical band heterotopia, Walker-Warburg syndrome, Alzheimer's disease epilepsy, posttraumatic epilepsy, progressive myoclonus epilepsy, reflex epilepsy, Rasmussen's syndrome, temporal lobe epilepsy, limbic epilepsy, status epilepticus, abdominal epilepsy, massive bilateral myoclonus, catamenial epilepsy, Jacksonian seizure disorder, Unverricht-Lundborg disease, and photosensitive epilepsy. According to some embodiments, the individual has Dravet syndrome (DS). In certain embodiments, the individual has an epilepsy caused by a genetic mutation. In some embodiments, the individual has an epilepsy having a non-genetic etiology, non-limiting examples of which include epilepsies caused by concussion, brain injury, and/or the like. According to some embodiments, the individual has been diagnosed as having epilepsy based on the individual having had two or more unprovoked seizures. The individual's epilepsy may include generalized seizures or partial (e.g., focal) seizures.

A variety of individuals are treatable according to the subject methods. Generally such individuals are "mammals" or "mammalian," where these terms are used broadly to describe organisms which are within the class mammalia, including the orders carnivore (e.g., dogs and cats), rodentia (e.g., mice, guinea pigs, and rats), and primates (e.g., humans, chimpanzees, and monkeys). In some embodiments, the individual is a human.

By "treat" or "treatment" is meant at least an amelioration of the symptoms associated with the pathological condition afflicting the individual, where amelioration is used in a broad sense to refer to at least a reduction in the magnitude of a parameter, e.g., symptom, associated with the pathological condition being treated, such as disease or disorder associated with PDE4 activity (e.g., epilepsy), where inhibiting PDE4 activity in the individual is beneficial. As such, treatment also includes situations where the pathological condition (e.g., epilepsy), or at least symptoms associated therewith, are completely inhibited, e.g., prevented from happening, or stopped, e.g. terminated, such that the individual no longer suffers from the pathological condition, or at least the symptoms that characterize the pathological condition. In some embodiments, an individual having epilepsy is treated by the present methods when one or more administrations of the PDE4 inhibitor results in the frequency and/or severity of seizures experienced by the individual being 90% or less, 80% or less, 70% or less, 60% or less, 50% or less, 40% or less, 30% or less, 20% or less, 10% or less, 5% or less, or 1% or less, as compared to the frequency and/or severity of seizures experienced by the individual in the absence of the one or more administrations of the PDE4 inhibitor.

Dosing is dependent on severity and responsiveness of the disease state to be treated. Optimal dosing schedules can be calculated from measurements of PDE4 inhibitor accumulation in the body of the individual. The administering physician can determine optimum dosages, dosing methodologies and repetition rates. Optimum dosages may vary depending on the relative potency of the PDE4 inhibitor, and can generally be estimated based on EC₅₀s found to be effective in *in vitro* and *in vivo* animal models, etc. In general, dosage is from 0.01 µg to 100 g per kg of body weight, and may be given once or more daily, weekly, monthly or yearly. The treating physician can estimate repetition rates for dosing based on measured residence times and concentrations of the PDE4 inhibitor in bodily fluids or tissues. Following successful treatment, it may be desirable to have the subject undergo maintenance therapy to prevent the recurrence of the disease state, where the PDE4 inhibitor is administered in maintenance doses, once or more daily, to once every several months, once every six months, once every year, or at any other suitable frequency.

The therapeutic methods of the present disclosure may include administering a single type of PDE4 inhibitor to an individual, or may include administering two or more types of PDE4 inhibitors to an individual, e.g., a cocktail of different PDE4 inhibitors.

In certain aspects, a PDE4 inhibitor is administered to the individual in combination with a second therapeutic agent as part of a combination therapy. For example, the methods of the present disclosure may include administering to an individual having epilepsy a therapeutically effective amount of a PDE4 inhibitor in combination with a second anti-epileptic drug. Non-limiting examples of a second anti-epileptic drug which may be used in combination with a PDE4 inhibitor include acetazolamide, diazepam, benzodiazepine, cannabadiol, carbamazepine, clobazam, clonazepam, eslicarbazepine acetate, ethosuximide, ethotoin, felbamate, fenfluramine, fosphenytoin, gabapentin, ganaxolone, huperzine A, lacosamide, lamotrigine, levetiracetam, lorazepam, nitrazepam, oxcarbazepine, perampanel, piracetam, phenobarbital, phenytoin, potassium bromide, pregabalin, primidone, retigabine, rufinamide, sodium valproate, stiripentol, tiagabine, topiramate, valproic acid, vigabatrin, or zonisamide. According to embodiments in which a PDE4 inhibitor and a second therapeutic agent are administered in combination, the PDE4 inhibitor and the second therapeutic agent may be administered concurrently or sequentially.

The PDE4 inhibitor may be administered to the individual using any available method and route suitable for drug delivery, including *in vivo* and *ex vivo* methods, as well as systemic and localized routes of administration. Conventional and pharmaceutically acceptable routes of administration include intranasal, intramuscular, intra-tracheal, subcutaneous, intradermal, topical application, ocular, intravenous, intra-arterial, nasal, oral, and other enteral and parenteral routes of administration. Routes of administration may be combined, if desired, or adjusted depending upon the PDE4 inhibitor and/or the desired effect. The PDE4 inhibitor may be administered in a single dose or in multiple doses. In some embodiments, the PDE4 inhibitor is administered by oral, parenteral, intranasal, intrathecal, intracranial, or transdermal administration. In some embodiments, the PDE4 inhibitor is administered orally. In some embodiments, the PDE4 inhibitor is administered locally. In some embodiments, the PDE4 inhibitor is administered ocularly. In some embodiments, the PDE4 inhibitor is administered intracranially. In some embodiments, the PDE4 inhibitor is administered intravenously. In some embodiments, the PDE4 inhibitor is administered by injection, e.g., for systemic delivery (e.g., intravenous infusion) or to a local site. In some embodiments, the PDE4 inhibitor is administered via an inhalational route. In some embodiments, the PDE4 inhibitor is administered intranasally. In some embodiments, the PDE4 inhibitor does not readily cross the blood-brain barrier (BBB), and the PDE4 inhibitor is administered intranasally to bypass the BBB. Further details regarding bypassing the BBB by intranasal administration may be found, e.g., in Mohanty et al. (2015) Curr Pharm Des. 21(31):4606-13, and elsewhere.

### METHODS OF IDENTIFYING ANTI-EPILEPTIC AGENTS

Described herein, but not falling within the subject matter of the present invention are methods of identifying anti-epileptic agents. The methods of identifying an anti-epileptic agent include contacting a PDE4 polypeptide with a candidate agent in a PDE4 activity assay, where inhibition of activity of the PDE4 polypeptide by the candidate agent identifies the candidate agent as an anti-epileptic agent.

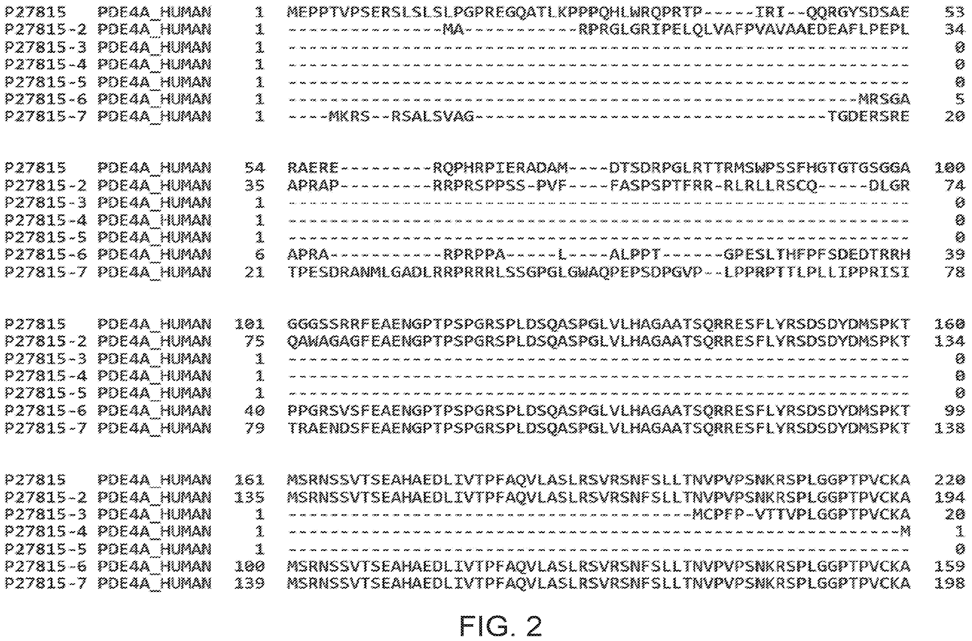

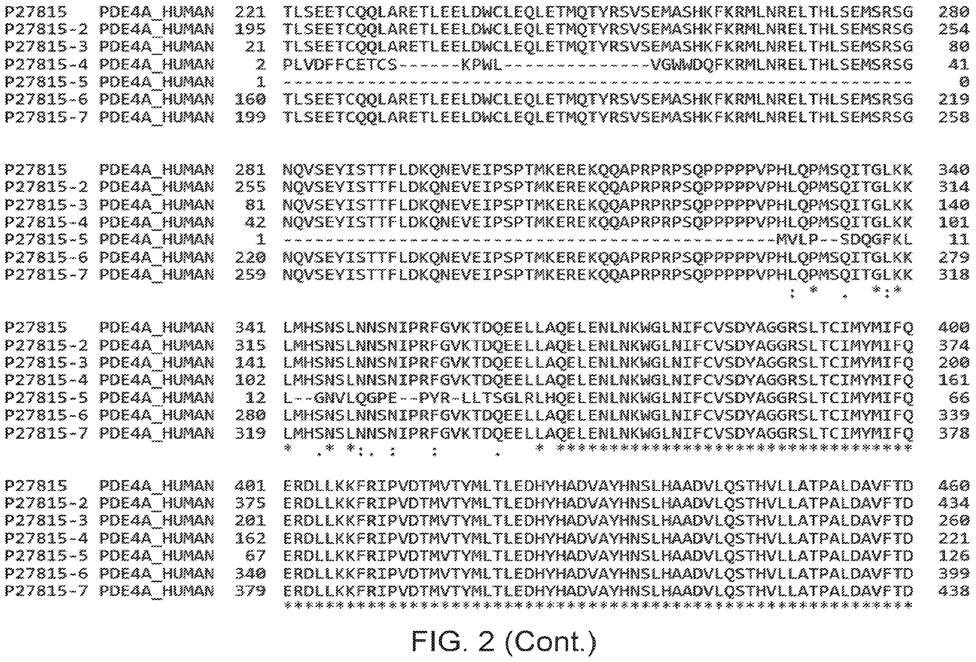

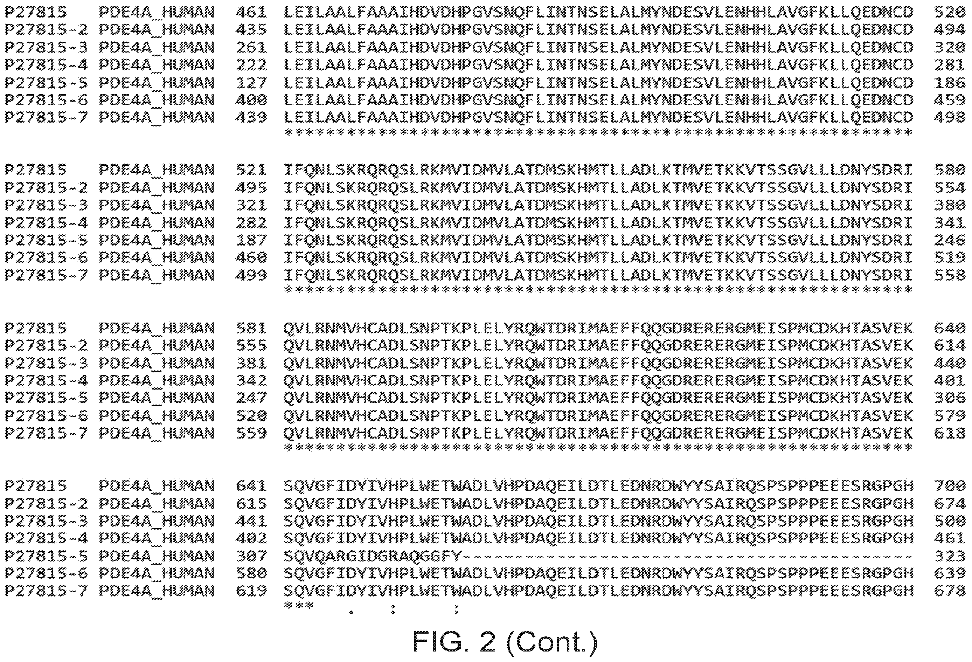

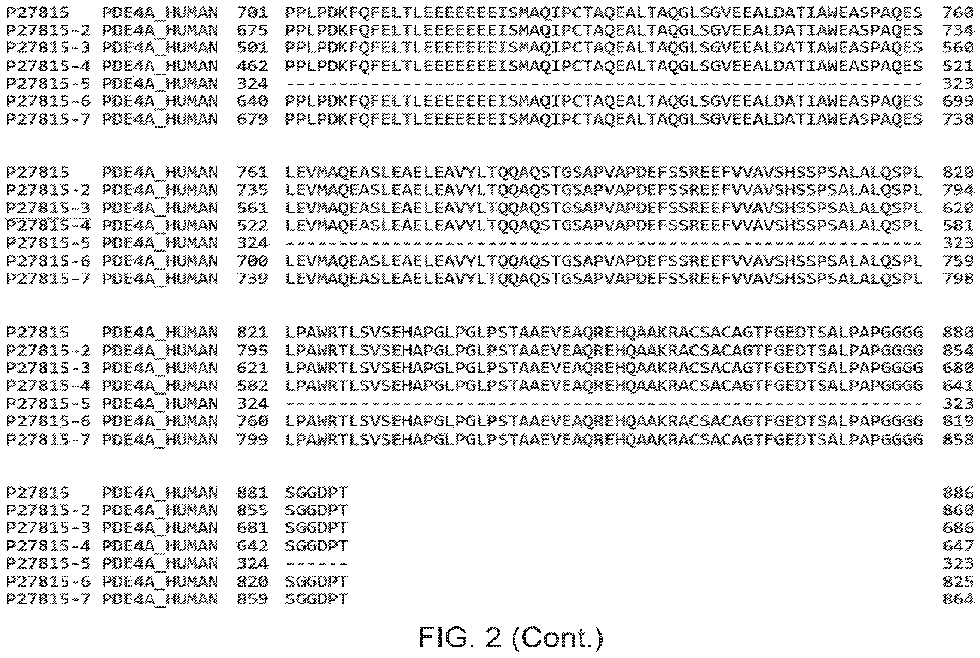

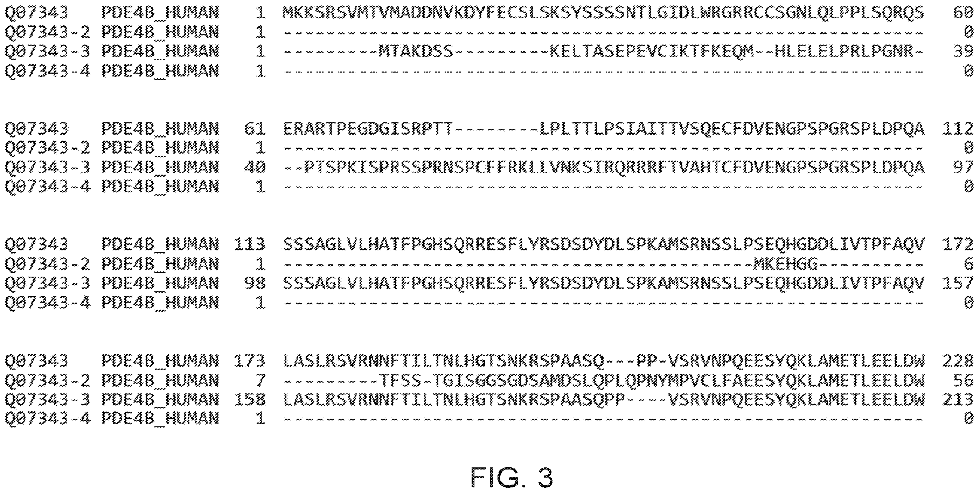

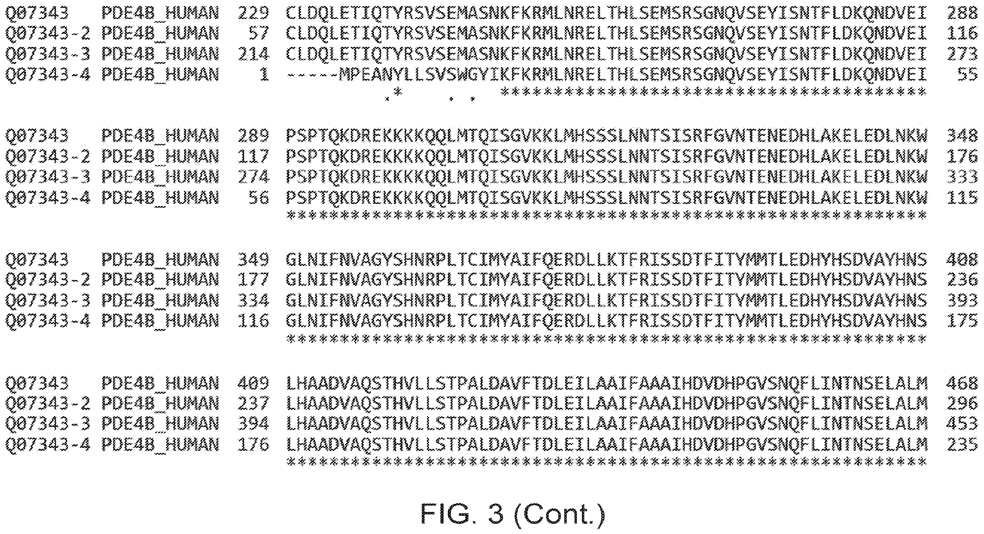

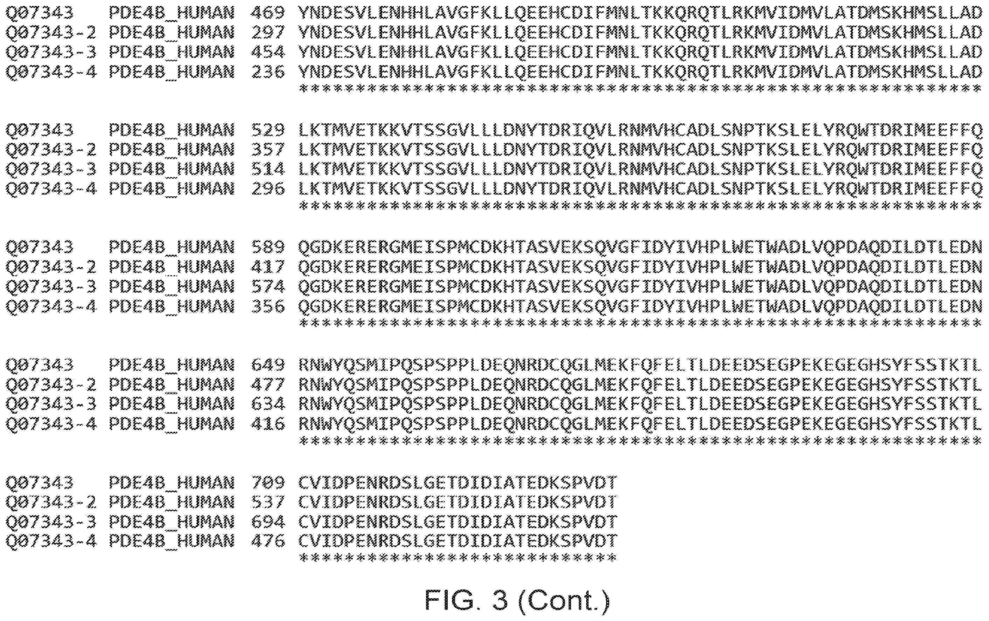

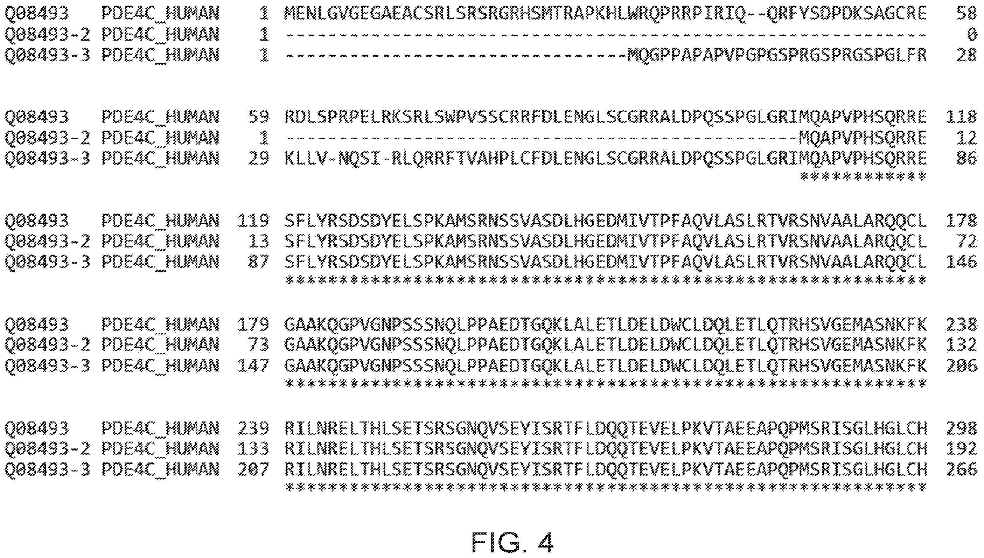

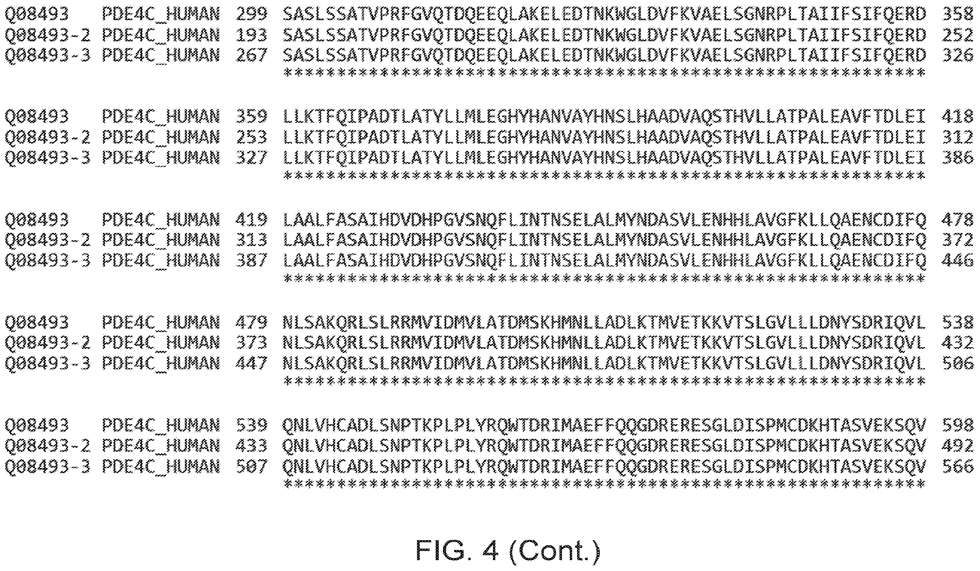

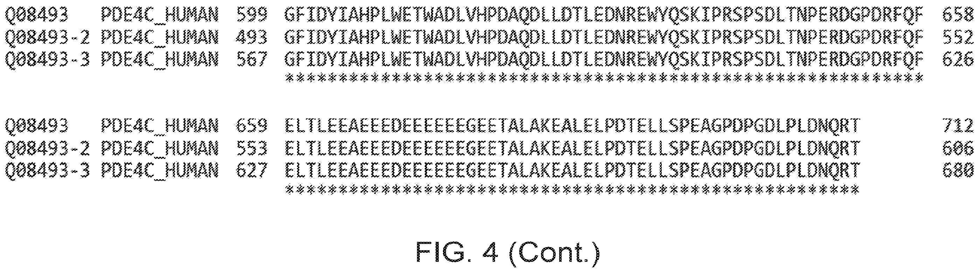

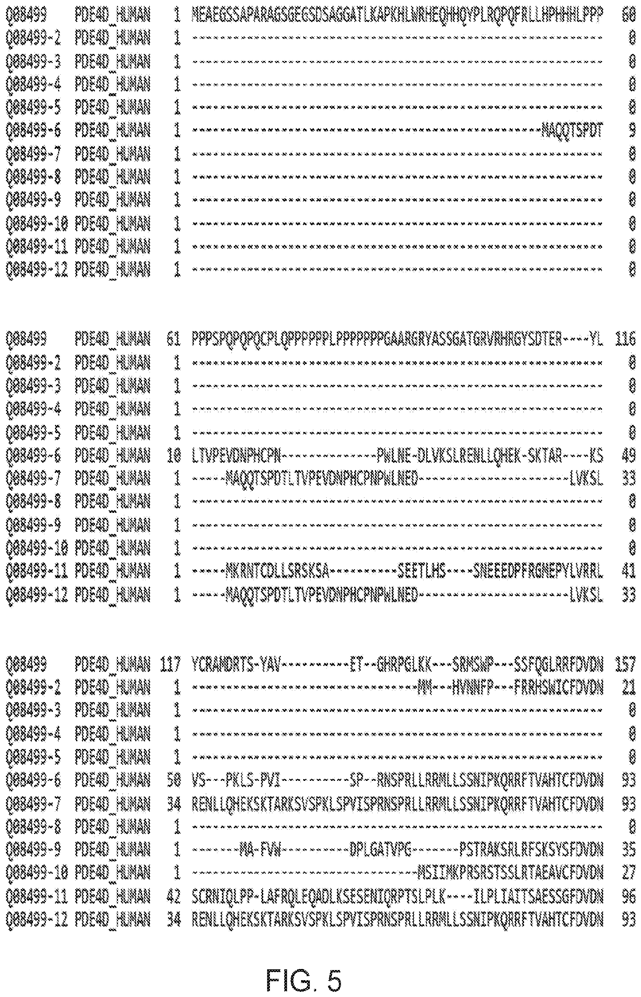

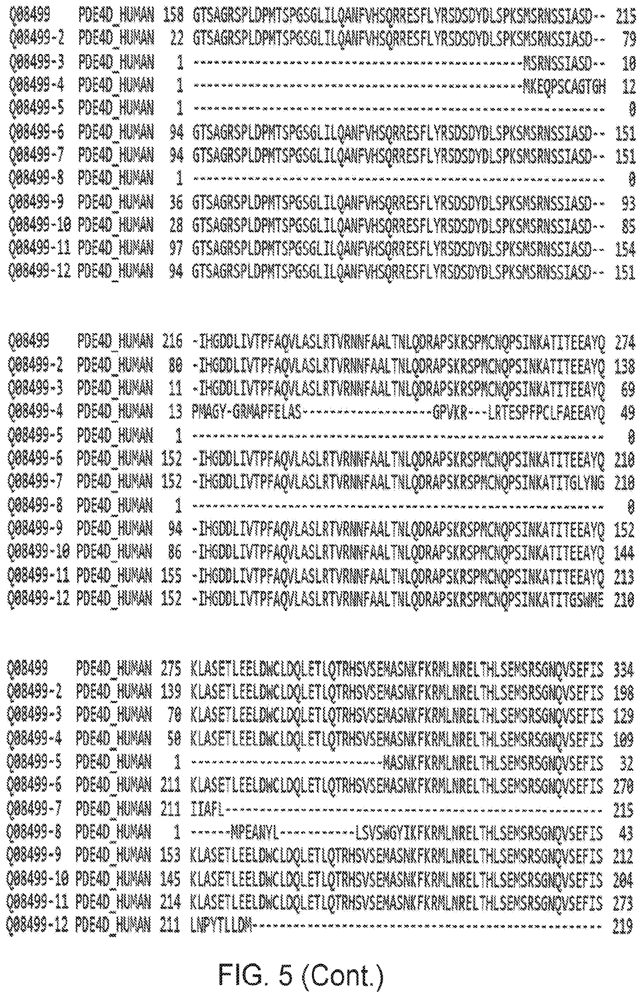

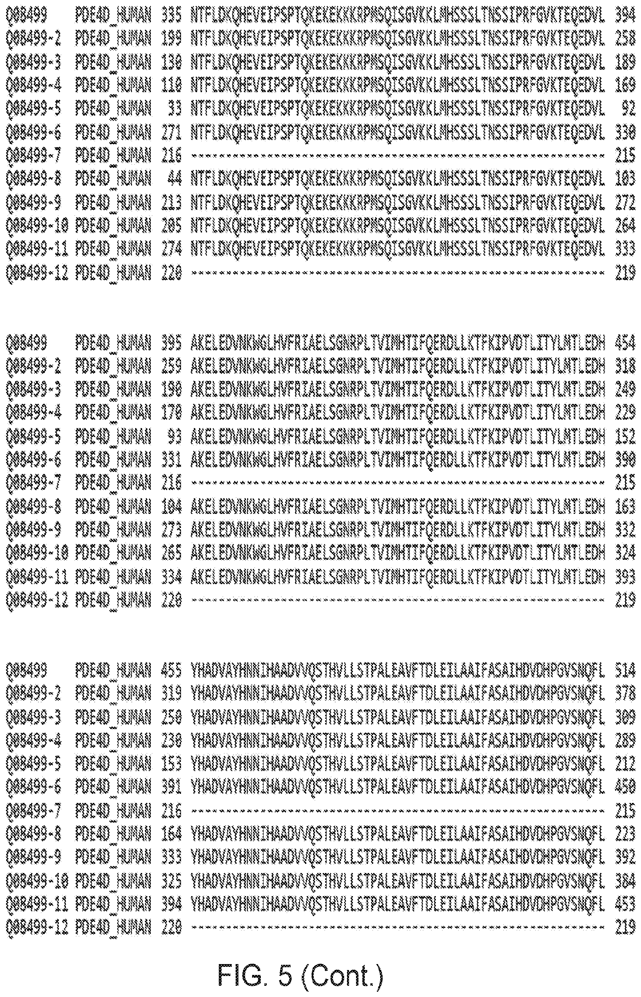

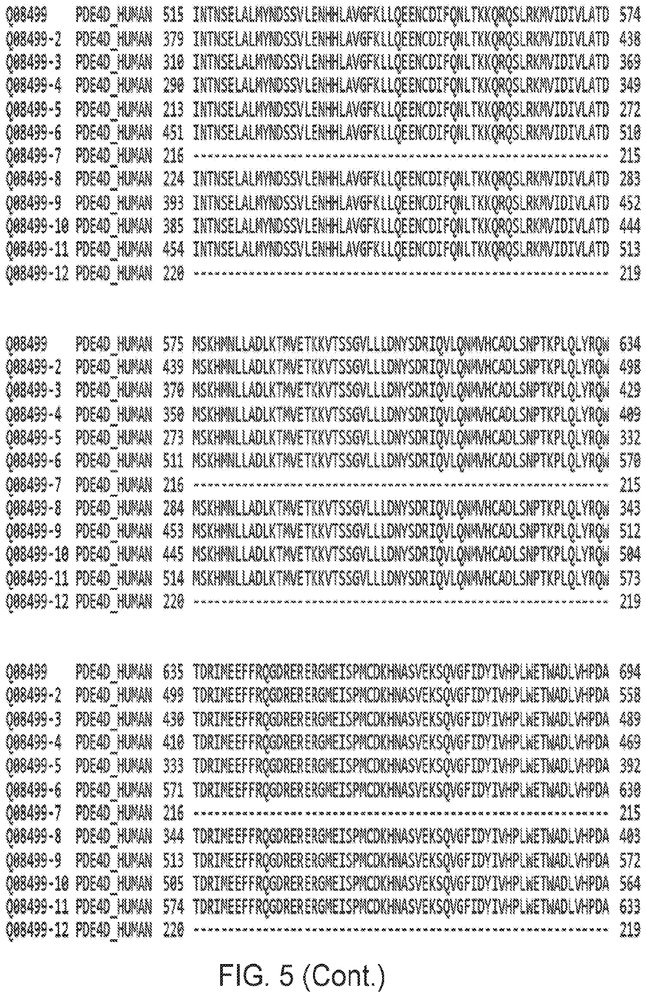

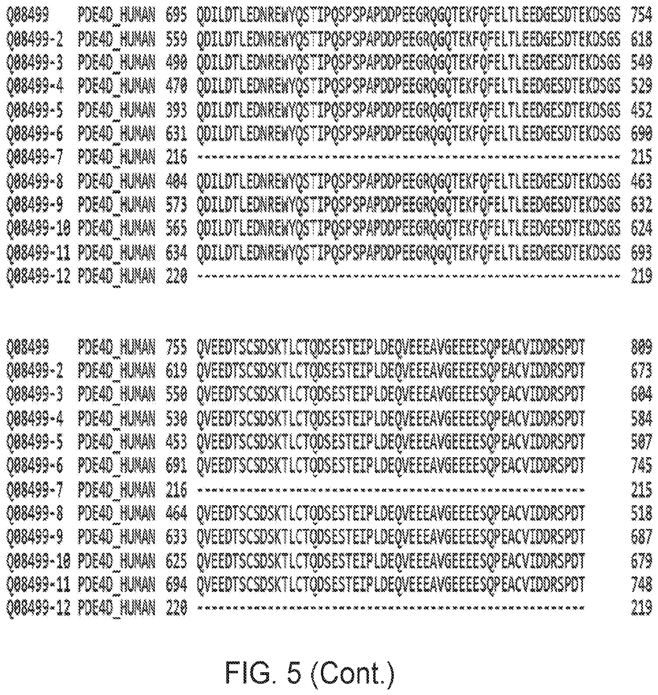

The PDE4 polypeptide contacted with the candidate agent may be any PDE4 polypeptide of interest. In some instances, the PDE4 polypeptide is PDE4A, PDE4B, PDE4C, or PDE4D, including any PDE4 polypeptide provided in FIGs. 2-5 or Table 1 above, or a functional variant thereof having phosphodiesterase activity. By "functional variant" is meant a PDE4 polypeptide having phosphodiesterase activity that includes one or more amino acid substitutions, deletions, insertions, or combination thereof, relative to the corresponding wild-type PDE4 polypeptide. For example, a functional variant of PDE4B may be a PDE4B polypeptide that includes one or more amino acid substitutions, deletions, insertions, or combination thereof, relative to wild-type PDE4B. A functional variant may include 70% or greater, 75% or greater, 80% or greater, 85% or greater, 90% or greater, 95% or greater, or 99% or greater, amino acid sequence homology or identity across 40% or greater, 50% or greater, 60% or greater, 70% or greater, 80% or greater, 90% or greater, or 95% or greater, of the length of the corresponding wild-type PDE4 polypeptide. The PDE4 polypeptide or functional variant thereof may be fused to a heterologous domain. Non-limiting examples of heterologous domains include linker domains (e.g., a glycine-serine linker, or other suitable linker domain), domains that find use in detecting the PDE4 polypeptide (e.g., a luciferase domain, or other suitable detectable domain), domains that find use in purifying the PDE4 polypeptide (e.g., a HIS tag, or other suitable purification tag), and/or the like.

The PDE4 activity assay may be performed using any suitable reagents (e.g., PDE4 substrates, etc.) and formats for assaying phosphodiesterase activity. In some instances, the PDE4 activity assay includes detecting the cleavage of cAMP or cGMP by the PDE4 polypeptide. Detection of PDE4 phosphodiesterase activity may be colorimetric-based, luminescence-based, fluorescence-based, radioactivity-based, and/or the like. In some instances, the assay is performed in a single tube, single well, multi-tube, multi-well (e.g., 24-well, 48-well, 96-well, 384-well, or other well format), or other suitable format. PDE4 activity assays are amenable to high-throughput formats such that large numbers of candidate agents (e.g., small molecules of a small molecule library) may be readily contacted with PDE4 polypeptides in parallel, e.g., separate wells.

In some instances, in the methods of identifying anti-epileptic agents, the contacting includes combining the PDE4 polypeptide and the candidate agent in a cell-free PDE4 activity assay. Cell-free assay reagents and kits for assessing phosphodiesterase activity and inhibition thereof that may be employed when practicing the methods of the present disclosure are known and include the PDE Activity Assay Kit ( 96-well colorimetric) available from Abcam, the PDE-Glo^{™} Phosphodiesterase Assay Kit (1-tube to 1536-well luminescence) available from Promega, the PDEase Kit available from FabGennix, the Bridge-It^{®} cAMP-Phosphodiesterase Assay Kit available from Mediomics, and the like. Additional approaches for assessing phosphodiesterase activity and inhibition thereof in cell-free format are described, e.g., in Rybalkin et al. (2013) Methods Mol Biol. 1020:51-62.

In some instances, in the methods of identifying anti-epileptic agents, the contacting includes combining the PDE4 polypeptide and the candidate agent in a cell-based PDE4 activity assay. By "cell-based" is meant the PDE4 polypeptide and the candidate agent are contacted within a cell. In some instances, the PDE4 polypeptide is expressed by the cell in which the contacting occurs. Cell-based assay reagents and kits for assessing phosphodiesterase activity and inhibition thereof that may be employed when practicing the methods of the present disclosure are known and include the ACTOne PDE Assay Kit available from eEnzyme, the Cell-Based PDE Assay Kit available from SB Drug Discovery, the K927-Total Phosphodiesterase Activity Assay Kit available from Biovision, and the like. Additional approaches for assessing phosphodiesterase activity and inhibition thereof in cell-free format are described, e.g., in Titus et al. (2008) J Biomol Screen. 13(7):609-618 (describing a cell-based PDE4 assay in 1536-well plate format for high throughput screening involving a constitutively active GPCR as a driving force for cAMP production and a cyclic nucleotide gated (CNG) cation channel as a biosensor in 1536-well plates); Allen et al. (1999) Biochem Pharmacol. 57(12):1375-82; and Qiu et al. (2003) Eur J Pharmacol. 472(1-2):73-80*.*

In some instances, the PDE4 activity assay further includes contacting the PDE4 polypeptide with a positive control agent known to inhibit PDE4 activity. Non-limiting examples of positive control agents that may be employed include AN2728, drotaverine, ibudilast, irsogladine, piclamilast, roflumilast, rolipram, theophylline, apremilast, and any combination thereof.

The candidate agent may be any type of candidate agent of interest. In some instances, the candidate agent is a small molecule. In some instances, the small molecule was selected as a candidate agent based on an *in silico* screen for PDE4 inhibitors. The *in silico* screen may have been a screen for a PDE4 inhibitor that is selective among PDE4A, PDE4B, PDE4C, and PDE4D. For example, the *in silico* screen may have been an *in silico* screen for a PDE4 inhibitor that selectively inhibits PDE4A, PDE4B, and/or PDE4C (e.g., versus PDE4D). In some instances, the small molecule was selected as a candidate agent based on an *in silico* screen for inhibitors that are selective for PDE4B versus PDE4D. Accordingly, in some instances, the methods may further include - when the candidate agent is determined to inhibit activity of the PED4 polypeptide - determining whether the candidate agent exhibits selective inhibition among PDE4A, PDE4B, PDE4C, and PDE4D.

In some instances, when the candidate agent is a small molecule, the small molecule is part of a library of small molecule candidate agents. In some instances, the methods include contacting a library of small molecule candidate agents with PDE4 polypeptides in high-throughput format, e.g., 96-well, 384-well, 1536-well, or other high throughput format.

### COMPOSITIONS

Described herein, but not falling within the subject matter of the present invention are compositions. In some instances, the compositions find use, e.g., in practicing the methods of the present disclosure.

In some instances, a composition of the present disclosure includes any of the PDE4 inhibitors described elsewhere herein, including any anti-epileptic agent identified by the methods of identifying anti-epileptic agents of the present disclosure.

In certain instances, a composition of the present disclosure includes the PDE4 inhibitor (and optionally, a second anti-epileptic agent) present in a liquid medium. The liquid medium may be an aqueous liquid medium, such as water, a buffered solution, or the like. One or more additives such as a salt (e.g., NaCl, MgCl₂, KCI, MgSO₄), a buffering agent (a Tris buffer, N-(2-Hydroxyethyl)piperazine-N'-(2-ethanesulfonic acid) (HEPES), 2-(N-Morpholino)ethanesulfonic acid (MES), 2-(N-Morpholino)ethanesulfonic acid sodium salt (MES), 3-(N-Morpholino)propanesulfonic acid (MOPS), N-tris[Hydroxymethyl]methyl-3-aminopropanesulfonic acid (TAPS), etc.), a solubilizing agent, a detergent (e.g., a non-ionic detergent such as Tween-20, etc.), a nuclease inhibitor, a protease inhibitor, glycerol, a chelating agent, and the like may be present in such compositions.

Pharmaceutical compositions are also provided. The pharmaceutical compositions of the present disclosure include a PDE4 inhibitor and a pharmaceutically acceptable carrier. Any pharmaceutical composition of the present disclosure may include - in addition to the PDE4 inhibitor - a second anti-epileptic agent. For example, provided are pharmaceutical compositions that include a PDE4 inhibitor and a second anti-epileptic agent. Non-limiting examples of second anti-epileptic agents which may be provided in a pharmaceutical composition of the present disclosure include acetazolamide, diazepam, benzodiazepine, cannabadiol, carbamazepine, clobazam, clonazepam, eslicarbazepine acetate, ethosuximide, ethotoin, felbamate, fenfluramine, fosphenytoin, gabapentin, ganaxolone, huperzine A, lacosamide, lamotrigine, levetiracetam, lorazepam, nitrazepam, oxcarbazepine, perampanel, piracetam, phenobarbital, phenytoin, potassium bromide, pregabalin, primidone, retigabine, rufinamide, sodium valproate, stiripentol, tiagabine, topiramate, valproic acid, vigabatrin, or zonisamide.

The PDE4 inhibitor (and optionally, a second anti-epileptic agent) can be incorporated into a variety of formulations for administration to an individual. More particularly, the PDE4 inhibitor can be formulated into pharmaceutical compositions by combination with appropriate, pharmaceutically acceptable excipients or diluents, and may be formulated into preparations in solid, semi-solid, liquid or gaseous forms, such as tablets, capsules, powders, granules, ointments, solutions, injections, inhalants and aerosols.

Formulations of the PDE4 inhibitor suitable for administration to an individual (e.g., suitable for human administration) are generally sterile and may further be free of detectable pyrogens or other contaminants contraindicated for administration to an individual according to a selected route of administration.

In pharmaceutical dosage forms, the PDE4 inhibitor can be administered alone or in appropriate association, as well as in combination, with a pharmaceutically active compound, e.g., a second anti-epileptic agent. The following methods and carriers/excipients are merely examples and are in no way limiting.

For oral preparations, the PDE4 inhibitor can be used alone or in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

The PDE4 inhibitor can be formulated for parenteral (e.g., intravenous, intra-arterial, intraosseous, intramuscular, intracerebral, intracerebroventricular, intracranial, intrathecal, subcutaneous, etc.) administration. In some embodiments, the PDE4 inhibitor is formulated for oral, parenteral, intranasal, intrathecal, intracranial, intracerebral, intracerebroventricular, or transdermal administration. In some embodiments, the PDE4 inhibitor is formulated for injection by dissolving, suspending or emulsifying the PDE4 inhibitor in an aqueous or non-aqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives.

Pharmaceutical compositions that include the PDE4 inhibitor may be prepared by mixing the PDE4 inhibitor having the desired degree of purity with optional physiologically acceptable carriers, excipients, stabilizers, surfactants, buffers and/or tonicity agents. Acceptable carriers, excipients and/or stabilizers are nontoxic to recipients at the dosages and concentrations employed, and include buffers such as phosphate, citrate, and other organic acids; antioxidants including ascorbic acid, glutathione, cysteine, methionine and citric acid; preservatives (such as ethanol, benzyl alcohol, phenol, m-cresol, p-chlor-m-cresol, methyl or propyl parabens, benzalkonium chloride, or combinations thereof); amino acids such as arginine, glycine, ornithine, lysine, histidine, glutamic acid, aspartic acid, isoleucine, leucine, alanine, phenylalanine, tyrosine, tryptophan, methionine, serine, proline and combinations thereof; monosaccharides, disaccharides and other carbohydrates; low molecular weight (less than about 10 residues) polypeptides; proteins, such as gelatin or serum albumin; chelating agents such as EDTA; sugars such as trehalose, sucrose, lactose, glucose, mannose, maltose, galactose, fructose, sorbose, raffinose, glucosamine, N-methylglucosamine, galactosamine, and neuraminic acid; and/or non-ionic surfactants such as Tween, Brij Pluronics, Triton-X, or polyethylene glycol (PEG).

The pharmaceutical composition may be in a liquid form, a lyophilized form or a liquid form reconstituted from a lyophilized form, wherein the lyophilized preparation is to be reconstituted with a sterile solution prior to administration. The standard procedure for reconstituting a lyophilized composition is to add back a volume of pure water (typically equivalent to the volume removed during lyophilization); however solutions comprising antibacterial agents may be used for the production of pharmaceutical compositions for parenteral administration.

An aqueous formulation of the PDE4 inhibitor may be prepared in a pH-buffered solution, e.g., at pH ranging from about 4.0 to about 7.0, or from about 5.0 to about 6.0, or alternatively about 5.5. Examples of buffers that are suitable for a pH within this range include phosphate-, histidine-, citrate-, succinate-, acetate-buffers and other organic acid buffers. The buffer concentration can be from about 1 mM to about 100 mM, or from about 5 mM to about 50 mM, depending, e.g., on the buffer and the desired tonicity of the formulation.

A tonicity agent may be included in the formulation to modulate the tonicity of the formulation. Example tonicity agents include sodium chloride, potassium chloride, glycerin and any component from the group of amino acids, sugars as well as combinations thereof. In some embodiments, the aqueous formulation is isotonic, although hypertonic or hypotonic solutions may be suitable. The term "isotonic" denotes a solution having the same tonicity as some other solution with which it is compared, such as physiological salt solution or serum. Tonicity agents may be used in an amount of about 5 mM to about 350 mM, e.g., in an amount of 100 mM to 350 mM.

A surfactant may also be added to the formulation to reduce aggregation and/or minimize the formation of particulates in the formulation and/or reduce adsorption. Example surfactants include polyoxyethylensorbitan fatty acid esters (Tween), polyoxyethylene alkyl ethers (Brij), alkylphenylpolyoxyethylene ethers (Triton-X), polyoxyethylene-polyoxypropylene copolymer (Poloxamer, Pluronic), and sodium dodecyl sulfate (SDS). Examples of suitable polyoxyethylenesorbitan-fatty acid esters are polysorbate 20, (sold under the trademark Tween 20^{™}) and polysorbate 80 (sold under the trademark Tween 80^{™}). Examples of suitable polyethylene-polypropylene copolymers are those sold under the names Pluronic^{®} F68 or Poloxamer 188^{™}. Examples of suitable Polyoxyethylene alkyl ethers are those sold under the trademark Brij^{™}. Example concentrations of surfactant may range from about 0.001% to about 1% w/v.

A lyoprotectant may also be added in order to protect the PDE4 inhibitor against destabilizing conditions during a lyophilization process. For example, known lyoprotectants include sugars (including glucose and sucrose); polyols (including mannitol, sorbitol and glycerol); and amino acids (including alanine, glycine and glutamic acid). Lyoprotectants can be included in an amount of about 10 mM to 500 nM.

In some instances, the pharmaceutical composition includes the PDE4 inhibitor, and one or more of the above-identified agents (e.g., a surfactant, a buffer, a stabilizer, a tonicity agent) and is essentially free of one or more preservatives, such as ethanol, benzyl alcohol, phenol, m-cresol, p-chlor-m-cresol, methyl or propyl parabens, benzalkonium chloride, and combinations thereof. In other instances, a preservative is included in the formulation, e.g., at concentrations ranging from about 0.001 to about 2% (w/v).

### KITS

Described herein, but not falling within the subject matter of the present invention are kits. In some instances, the kits find use, e.g., in practicing the methods of the present disclosure.

In some instances, a kit of the present disclosure includes any of the PDE4 inhibitors described elsewhere herein, including any anti-epileptic agent identified by the methods of identifying anti-epileptic agents of the present disclosure.

In some instances, a kit of the present disclosure includes a pharmaceutical composition including a PDE4 inhibitor and a pharmaceutically acceptable carrier. For example, provided are kits that include any of the pharmaceutical compositions of the present disclosure, including any of the pharmaceutical compositions described in the Compositions section hereinabove. In some instances, a kit of the present disclosure includes a pharmaceutical composition that - in addition to a PDE4 inhibitor - further includes a second anti-epileptic agent. For example, a kit that finds use in methods of treating epilepsy may include a pharmaceutical composition that includes a PDE4 inhibitor and a second anti-epileptic agent. In some instances, a kit of the present disclosure further includes a second anti-epileptic agent provided in a pharmaceutical composition separate from the pharmaceutical composition comprising the PDE4 inhibitor. Non-limiting examples of second anti-epileptic agents which may be provided in a kit of the present disclosure (in the same or different pharmaceutical composition as the PDE4 inhibitor) include acetazolamide, diazepam, benzodiazepine, cannabadiol, carbamazepine, clobazam, clonazepam, eslicarbazepine acetate, ethosuximide, ethotoin, felbamate, fenfluramine, fosphenytoin, gabapentin, ganaxolone, huperzine A, lacosamide, lamotrigine, levetiracetam, lorazepam, nitrazepam, oxcarbazepine, perampanel, piracetam, phenobarbital, phenytoin, potassium bromide, pregabalin, primidone, retigabine, rufinamide, sodium valproate, stiripentol, tiagabine, topiramate, valproic acid, vigabatrin, or zonisamide.

Kits for practicing the subject methods may include a quantity of the PDE4 inhibitor (and optionally, a second anti-epileptic agent), present in unit dosages, e.g., ampoules, or a multi-dosage format. As such, in certain instances, the kits may include one or more (e.g., two or more) unit dosages (e.g., ampoules) of a pharmaceutical composition that includes the PDE4 inhibitor (and optionally, a second anti-epileptic agent) and/or one or more (e.g., two or more) unit dosages (e.g., ampoules) of a pharmaceutical composition that includes a second anti-epileptic agent.

The term "unit dosage", as used herein, refers to physically discrete units suitable as unitary dosages for human and animal subjects, each unit containing a predetermined quantity of the composition calculated in an amount sufficient to produce the desired effect. The amount of the unit dosage depends on various factors, such as the particular PDE4 inhibitor employed, the effect to be achieved, and the pharmacodynamics associated with the PDE4 inhibitor, in the individual. In yet other instances, the kits may include a single multi dosage amount of a composition including the PDE4 inhibitor (and optionally, a second anti-epileptic agent).

Components of the kits may be present in separate containers, or multiple components may be present in a single container. For example, in a kit that includes both a PDE4 inhibitor and a second anti-epileptic agent, the PDE4 inhibitor and the second anti-epileptic agent may be provided in the same composition (e.g., in one or more containers) or may be provided in separate compositions in separate containers. Suitable containers include individual tubes (e.g., vials), ampoules, or the like.

A kit of the present disclosure may further include instructions. For example, a kit that includes a PDE4 inhibitor may include instructions for administering the PDE4 inhibitor to an individual in need thereof. In some instances, the instructions include instructions for administering the PDE4 inhibitor to an individual having epilepsy, including one or more of any type of epilepsy described elsewhere herein.

The instructions may be recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or sub-packaging) etc. In other instances, the instructions are present as an electronic storage data file present on a suitable computer readable storage medium, e.g., portable flash drive, DVD, CD-ROM, diskette, etc. In yet other instances, the actual instructions are not present in the kit, but means for obtaining the instructions from a remote source, e.g. via the internet, are provided. An example of this instance is a kit that includes a web address where the instructions can be viewed and/or from which the instructions can be downloaded. As with the instructions, the means for obtaining the instructions is recorded on a suitable substrate.

The following examples are offered by way of illustration and not by way of limitation.

### EXPERIMENTAL

### Example 1 - PDE4 inhibition restores bioenergetics to baseline levels in scn1lab and kcna1 mutant zebrafish

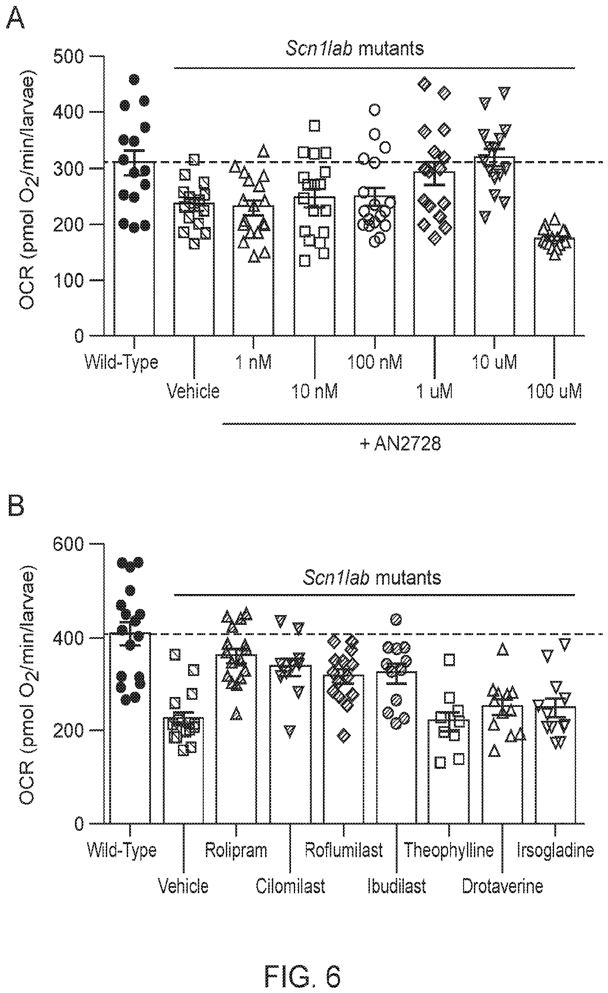

Shown in FIG. 6 (panel A) is data demonstrating that an example PDE4 inhibitor (AN2728) dose-dependently restores bioenergetics to baseline levels in *scn1lab* mutant zebrafish (in FIGs. 6-8, *"Scn1a"* mutant zebrafish are *scn1lab* mutant zebrafish). The *scn1lab* mutant zebrafish employed in this Experimental section are homozygous, loss-of-function mutants. To measure bioenergetics in whole, living zebrafish (further details of which may be found in lbhazehiebo et al. (2018) Brain 141(3):744-761), zebrafish larvae (5 days post-fertilization, dpf) were seeded into 24-well islet capture microplates (Agilent) and pre-exposed to vehicle or drug for 20 minutes prior to the start of the assay. Oxygen consumption rates (OCR) were measured using the Seahorse Bioanalyzer (Agilent). In this example, *scn1lab* mutant zebrafish larvae displayed depressed OCR relative to wild-type (WT) control, indicating that mitochondrial respiration is decreased in the *scn1lab* mutant background. In the presence of AN2728, a dose-dependent restoration of bioenergetics was observed, with 1µM and 10µM fully restoring OCR response to WT levels (FIG. 6, panel A). The 1nM-100nM doses were ineffective and the 100µM dose was toxic as demonstrated by the drop if OCR response below that of the mutant levels.

Shown in FIG. 6 (panel B) is data demonstrating that a variety of PDE4 inhibitors (in this example, Rolipram, Cilomilast, Roflumilast, Ibudilast, Theophylline, Drotaverine, and Irsogladine) are effective in restoring bioenergetics to baseline levels in *scn1lab* mutant zebrafish. In a similar assay to that described above, *scn1lab* mutants are exposed to a 40 µM dose of a range of PDE4 inhibitors. Rolipram, cilomilast, roflumilast, ibudilast all restored OCR response to baseline levels observed in the WT larvae, indicating that inhibition of PDE4 by these drugs reversed the decrease in bioenergetics demonstrated in the *scn1lab* mutant zebrafish when exposed to vehicle.

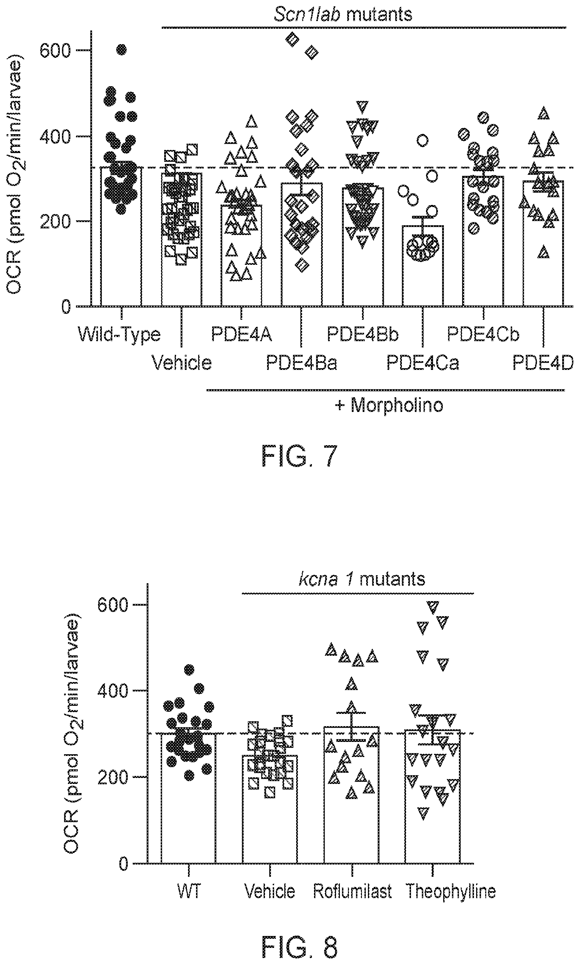

Shown in FIG. 7 is data demonstrating that PDE4B-, 4C- and 4D-morpholinos are effective in restoring bioenergetics to baseline levels in *scn1lab* mutant zebrafish. *scn1lab* mutant zebrafish embryos were injected at the one cell stage with an ATG- and/or a splice-blocking morpholino (Gene-Tools, Philomath, OR) designed to knockdown specific PDE4 isoforms in the genetic background of an epileptic zebrafish. Due to gene duplication in the zebrafish genome, many genes that have a single ortholog in humans have two isoforms in zebrafish. Morpholinos were designed to block each zebrafish isoform singly. Morpholino-injected *scn1lab* mutant zebrafish were sorted at 24hpf and viable embryos were seeded into islet microplates for bioenergetics assay (Seahorse bioanalyzer) at 5dpf. Morpholinos targeting PDE4Ba, PDE4Bb, PDE4Cb, and PDE4D restored OCR response to WT baseline levels in the epileptic background, indicating that knockdown of PDE4 signaling alleviates the decrease in mitochondrial function observed in the *scn1lab* mutants.

Shown in FIG. 8 is data demonstrating that two example PDE4 inhibitors (Roflumilast and Theophylline) restore bioenergetics in a *kcna1* mutant zebrafish back to baseline levels observed in a wild-type fish. *kcna1* mutants are a model for generalizable epilepsy and also sudden unexpected death in epilepsy (SUDEP), indicating that inhibiting PDE4 is likely to elicit effective anti-seizure properties across epilepsy conditions more broadly.

### Example 2 - PDE4 inhibition blocks seizure-like hyperexcitability to baseline levels in scn1lab zebrafish

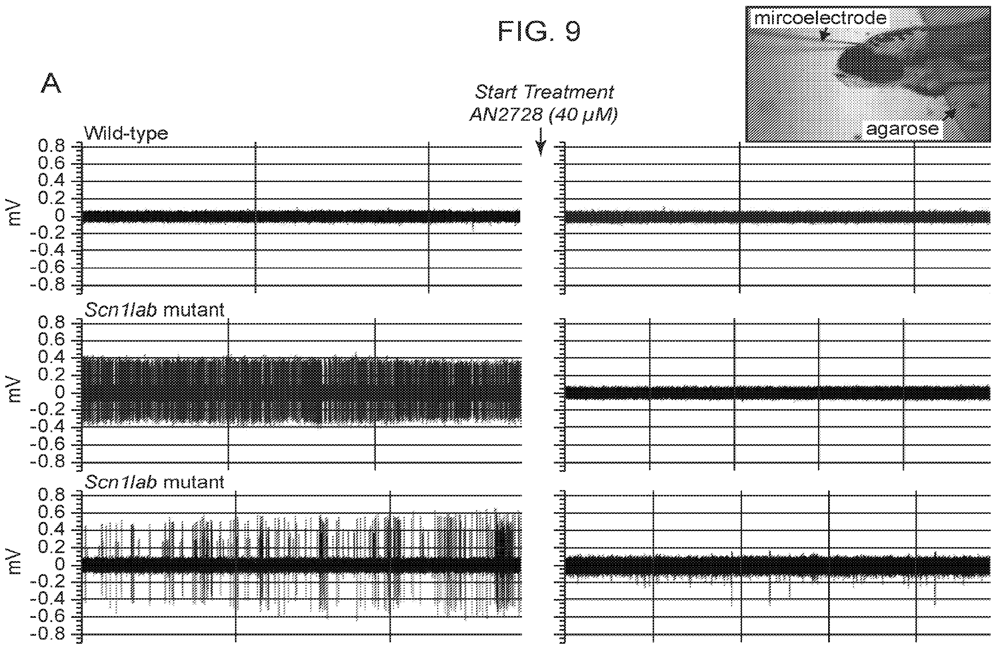

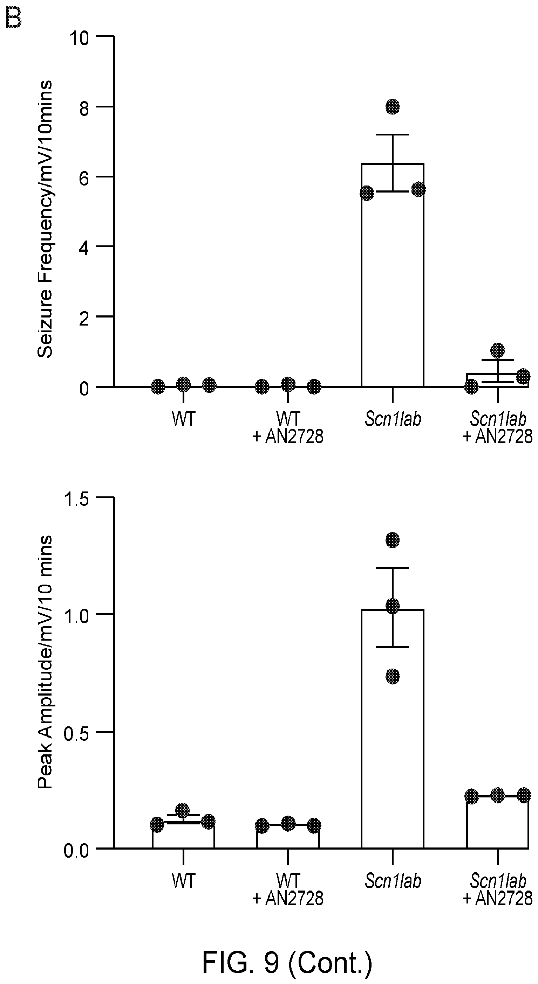

Shown in FIG. 9 is data demonstrating that an example PDE4 inhibitor (AN2728) blocks seizure-like hyperexcitability to baseline levels in *scn1lab* zebrafish. Briefly, 6 dpf zebrafish (WT and *scn1lab* mutants) were paralyzed (α-bungarotoxin, 1mg/ml, Tocris) and embedded in agarose. The dorsal side of the zebrafish was exposed to the agarose gel surface and a glass microelectrode was placed into the tectum opticum of zebrafish and electrophysiological measurements were recorded. After 20 min of baseline recording, drugs (final concentration 20 µM) were added directly to the embryo media and continued recordings in the same zebrafish were captured over the next 20 min. Care was taken not to disrupt the pipette or move the fish in any manner. Seizure-like activity was defined by high-frequency, large amplitude spikes, as defined elsewhere (Baraban et al, 2013). Both itcal (>1000 ms in duration) and inter-ictal (<300 ms in duration) activity was observed upon zooming in on a section of the EEG trace (data not shown), consistent with epileptiform events. Exposure to PDE inhibitor (AN2728, 40 µM) blocked this seizure-like activity to baseline levels. Quantification of these hyperexcitable events across multiple *scn1lab* mutant zebrafish (n=6-10) (FIG. 9, panel B) shows a decrease in seizure frequency and peak amplitude in the mutants following treatment with AN2728.

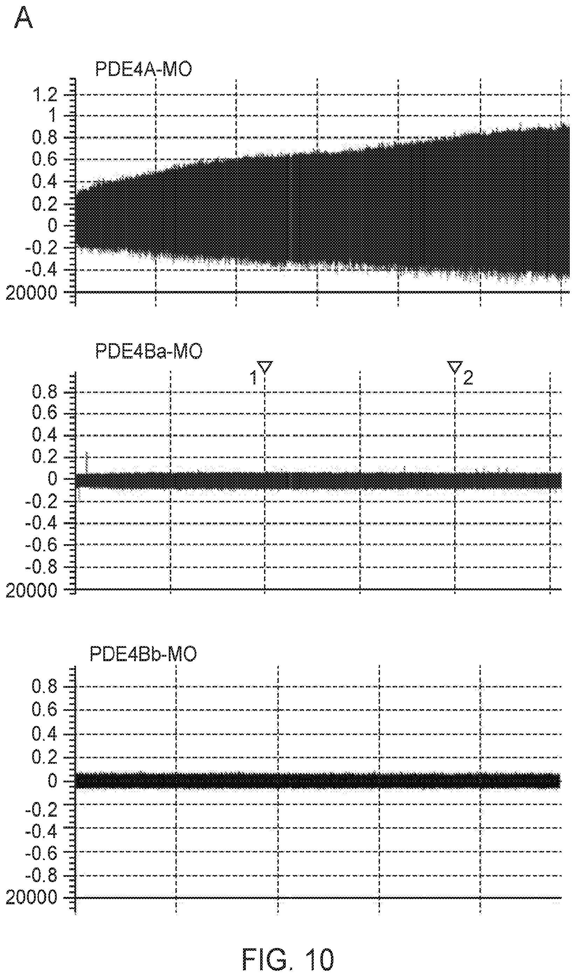

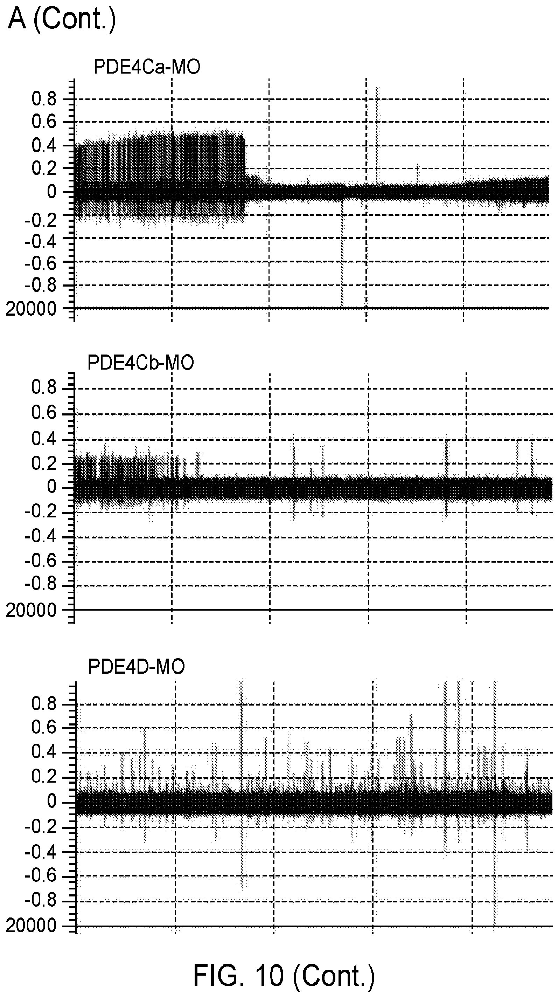

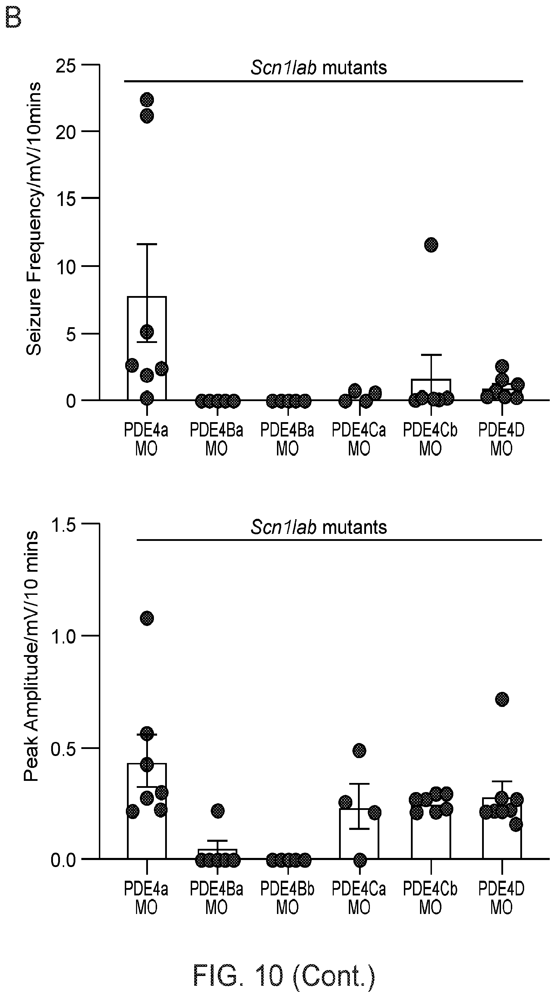

Shown in FIG. 10 is data demonstrating that a PDE4B morpholino is effective in blocking seizure-like hyperexcitability to baseline levels in *scn1lab* zebrafish. As described above, injection of PDE4 isoform-specific morpholinos targeting PDE4Ba and PDE4Bb blocked the hyperexcitable phenotype observed in *scn1lab* mutants. PDE4Ca-, PDE4Cb-, and PDE4D-morpholinos all partially blocked this seizure-like activity, whereas PDE4A-morpholino had no effect and perhaps even exacerbated the hyperexcitable phenotype. Quantification of these hyperexcitable events in *scn1lab* mutant zebrafish (n=6-10) that had been injected with a PDE4 isoform-targeting morpholino shows a decrease in seizure frequency and amplitude for PDE4B-, PDE4C-, and PDE4D-morpolinos when compared to *scn1lab* levels shown in FIG. 9, panel B.

### Example 3 - PDE4 inhibition decreases hyperexcitability in mouse Scn1a mutant brain ex vivo slices

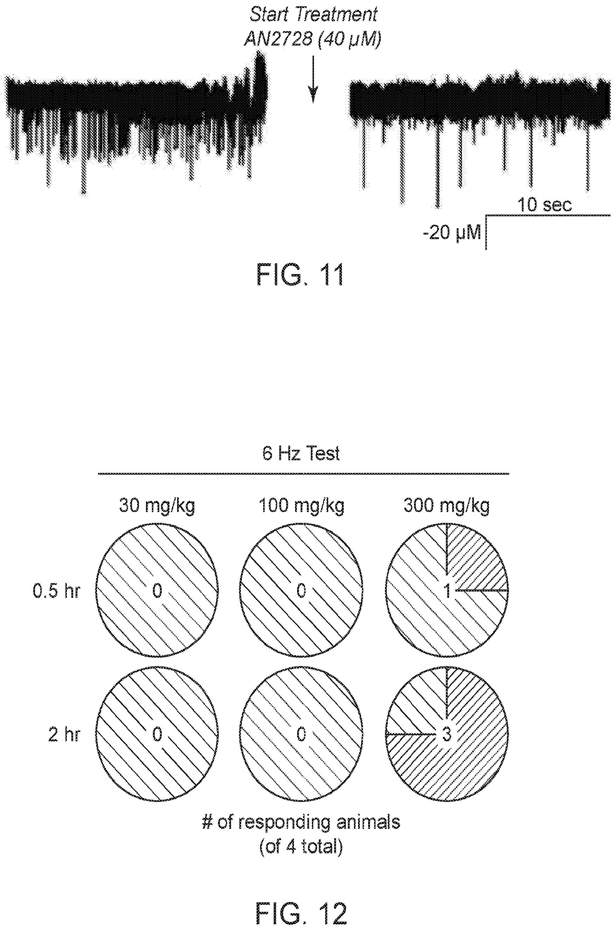

FIG. 11 provides EEG data demonstrating that an example PDE4 inhibitor (AN2728) decreases hyperexcitability in mouse *Scn1a* mutant brain *ex vivo* slices. The mouse *Scn1a* mutants employed in this Experimental section were heterozygous mutants (*Scn1a-*/*-* mice die early postnatally). As shown, EEG recordings in *Scn1a* mutant mouse *ex vivo* showed that AN2728 decreased a hyperexcitable phenotype (n = 3). In this assay, adult *Scn1a* mutant brains were rapidly removed and sectioned for electrophysiological assays. Here, extracellular field recordings were conducted to measure baseline electrophysiological activity, prior to the addition of PDE4 inhibitor (AN2728, 40 µM). A decrease in hyperexcitable amplitude and frequency was measured in the presence of AN2728.

### Example 4 - PDE4 inhibition protects against induction of seizures in a 6Hz-induction mouse model

Provided in FIG. 12 is data demonstrating that an example PDE4 inhibitor (AN2728) protects against induction of seizures in a 6Hz-induction mouse model. To examine the acute effect of PDE4 inhibition on seizures, the 6Hz psychomotor model that represents therapy-resistant limbic seizures was employed (White *et al.,* 1995; Barton *et al.,* 2001). Briefly, compounds were screened for their ability to block psychomotor seizures induced by a low-frequency (6 Hz), long-duration (3 sec) stimulus delivered through corneal electrodes. These seizures are believed to model partial seizures observed in humans. When the animals were dosed 30 min prior to the 6 Hz test, only 1 animal responded at the highest dose (300 mg/kg). However, when treated with AN2728 two hours prior to the 6 Hz test, three out of four animals responded.

### Example 5 - PDE4 inhibition protects against hyperthermia-induced seizures in Scn1a mutant mice

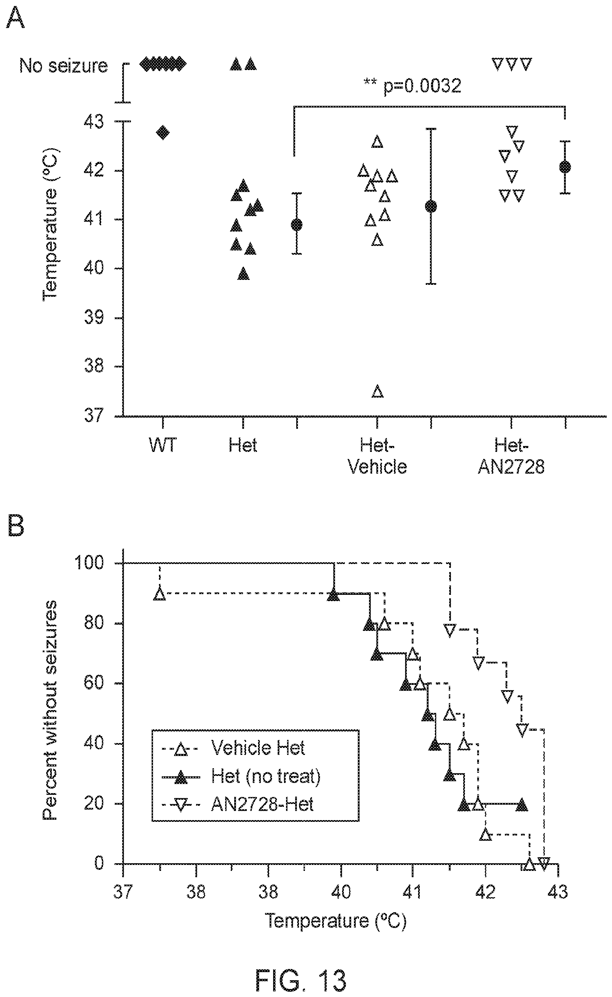

As shown in FIG. 13 (panels A and B), an example PDE4 inhibitor (AN2728) protects against hyperthermia-induced seizures in *Scn1a* mutant mice. In this model, a *Scn1a* mutant animal's core temperature is raised using an external heat source and consequently, brain temperature is raised and this leads to hyperthermic seizures. These hyperthermic seizures are thought to represent febrile seizures. In the *Scn1a* mutants, seizures begin around 41°C, whereas in the WT seizures are rarely detected even at temperatures as high as 43°C. Following treatment with a PDE4 inhibitor (AN2728), *Scn1a* mutants are protected against hyperthermia-induced seizures, whereas some animals do not ever experience a seizure (n=3) and the rest only experience a seizure at higher temperatures (e.g., 42°C). FIG12B is the same data graphed differently.

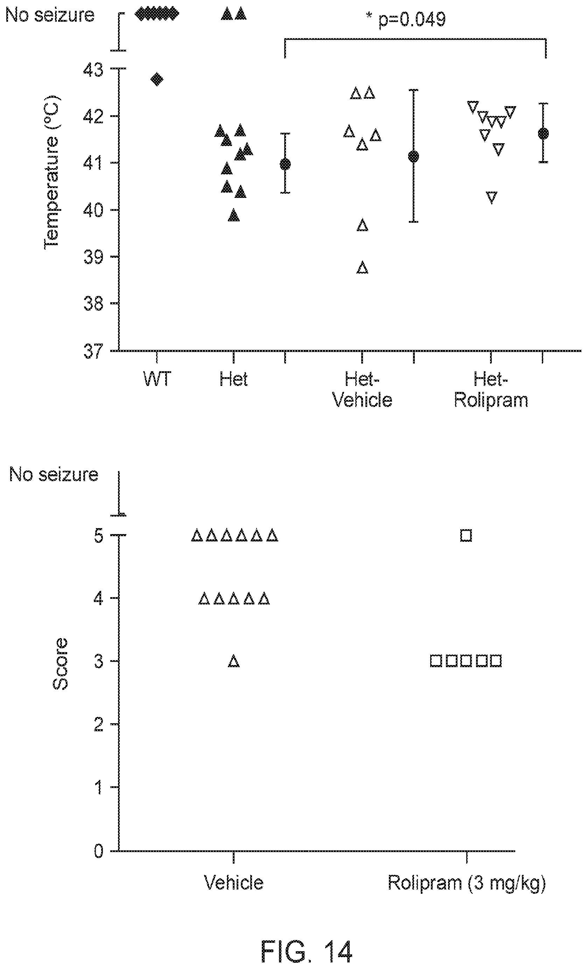

Shown in FIG. 14 is data demonstrating that a two-fold selective PDE4B inhibitor (rolipram) is effective in protecting against hyperthermia-induced seizures in *Scn1a* mutant mice. Using the hyperthermia-induction assay described above, treatment of *Scn1a* mutant mice with a different PDE4B inhibitor (rolipram, 3 mg/kg) was also neuroprotective against hyperthermia-induced seizures, raising not only the temperature required to induce a seizure (panel A) but also the severity of the seizure from mostly 4-5 level seizure to level 3, as measured using the Racine scale (panel B).

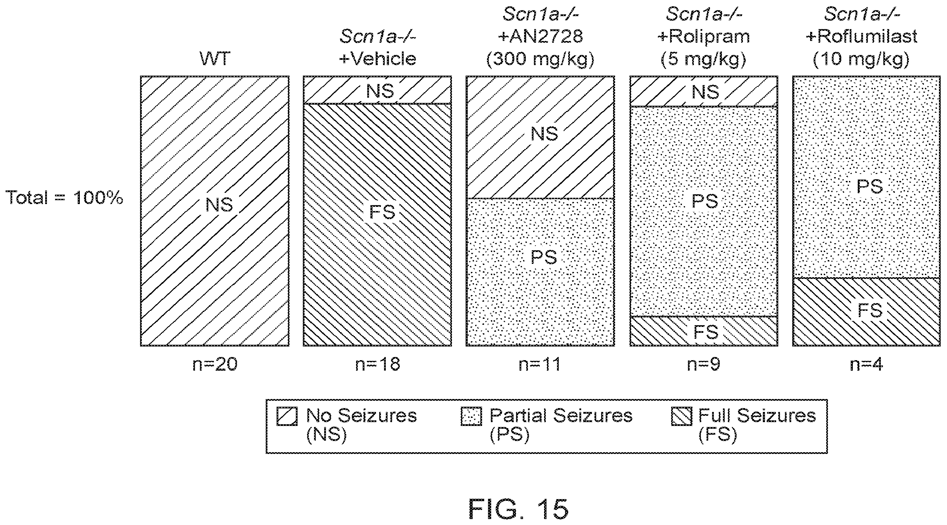

Shown in FIG. 15 is data comparing the effects of three example PDE4 inhibitors (AN2728, Rolipram, Roflumilast) in blocking seizures using the mouse *Scn1a* hyperthermia model described above. A partial block in seizures is noted when the temperature required to induce a seizure is statistically different than wildtype and a full block in seizures occurs when no seizure is elicited following pre-treatment with the PDE4 inhibitor.

## Claims

1. A phosphodiesterase 4 (PDE4) inhibitor for use in a method of treating epilepsy, wherein the method comprises administering to an individual having epilepsy a therapeutically effective amount of the PDE4 inhibitor, and wherein the PDE4 inhibitor is selective for PDE4B.

2. The PDE4 inhibitor that is selective for PDE4B for use according to claim 1, wherein the PDE4 inhibitor is a small molecule.

3. The PDE4 inhibitor that is selective for PDE4B for use according to claim 1 or 2, wherein the PDE4 inhibitor is an allosteric inhibitor.

4. The PDE4 inhibitor that is selective for PDE4B for use according to any one of claims 1 to 3, wherein the administering is by oral, parenteral, intranasal, intrathecal, intracranial, or transdermal administration.

5. The PDE4 inhibitor that is selective for PDE4B for use according to any one of claims 1 to 4, wherein the individual has an epilepsy selected from the group consisting of: benign Rolandic epilepsy, frontal lobe epilepsy, infantile spasms, juvenile myoclonic epilepsy (JME), juvenile absence epilepsy, childhood absence epilepsy, pyknolepsy, febrile seizures, progressive myoclonus epilepsy of Lafora, Lennox-Gastaut syndrome, Landau-Kleffner syndrome, Dravet syndrome (DS), Generalized Epilepsy with Febrile Seizures (GEFS+), Severe Myoclonic Epilepsy of Infancy (SMEI), Benign Neonatal Familial Convulsions (BFNC), West Syndrome, Ohtahara Syndrome, early myoclonic encephalopathy, migrating partial epilepsy, infantile epileptic encephalopathies, Tuberous Sclerosis Complex (TSC), focal cortical dysplasia, Type I Lissencephaly, Miller-Dieker Syndrome, Angelman's syndrome, Fragile X syndrome, epilepsy in autism spectrum disorders, subcortical band heterotopia, Walker-Warburg syndrome, Alzheimer's disease epilepsy, posttraumatic epilepsy, progressive myoclonus epilepsy, reflex epilepsy, Rasmussen's syndrome, temporal lobe epilepsy, limbic epilepsy, status epilepticus, abdominal epilepsy, massive bilateral myoclonus, catamenial epilepsy, Jacksonian seizure disorder, Unverricht-Lundborg disease, photosensitive epilepsy, epilepsy having a non-genetic etiology, epilepsy caused by concussion, and epilepsy caused by brain injury.

6. The PDE4 inhibitor that is selective for PDE4B for use according to any one of claims 1 to 5, wherein the individual has an epilepsy caused by a genetic mutation.

## Patentansprüche

1. Phosphodiesterase-4-Inhibitor (PDE4-Inhibitor) zur Verwendung in einem Verfahren zur Behandlung von Epilepsie, wobei das Verfahren die Verabreichung einer therapeutisch wirksamen Menge des PDE4-Inhibitors an eine Person mit Epilepsie umfasst, und wobei der PDE4-Inhibitor selektiv für PDE4B ist.

2. Für PDE4B selektiver PDE4-Inhibitor zur Verwendung nach Anspruch 1, wobei der PDE4-Inhibitor ein kleines Molekül ist.

3. Für PDE4B selektiver PDE4-Inhibitor zur Verwendung nach Anspruch 1 oder 2, wobei der PDE4-Inhibitor ein allosterischer Inhibitor ist.

4. Für PDE4B selektiver PDE4-Inhibitor zur Verwendung nach einem der Ansprüche 1 bis 3, wobei die Verabreichung oral, parenteral, intranasal, intrathekal, intrakraniell oder transdermal erfolgt.

5. Für PDE4B selektiver PDE4-Inhibitor zur Verwendung nach einem der Ansprüche 1 bis 4, wobei die Person an einer Epilepsie leidet, die aus der Gruppe ausgewählt ist, bestehend aus: benigner rolandischer Epilepsie, Frontallappenepilepsie, infantilen Spasmen, juveniler myoklonischer Epilepsie (JME), juveniler Absence-Epilepsie, kindlicher Absence-Epilepsie, Pyknolepsie, Fieberkrämpfen, progressiver Myoklonus-Epilepsie nach Lafora, Lennox-Gastaut-Syndrom, Landau-Kleffner-Syndrom, Dravet-Syndrom (DS), generalisierter Epilepsie mit Fieberkrämpfen (GEFS+), schwerer myoklonischer Epilepsie im Säuglingsalter (SMEI), benigner neonataler familiärer Krampfanfälle (BFNC), West-Syndrom, Ohtahara-Syndrom, früher myoklonischer Enzephalopathie, migrierender partieller Epilepsie, infantiler epileptischer Enzephalopathien, tuberöser Sklerose (TSC), fokaler kortikaler Dysplasie, Lissenzephalie Typ I, Miller-Dieker-Syndrom, Angelman-Syndrom, Fragile-X-Syndrom, Epilepsie bei Störungen des autistischen Spektrums, Subkortikalband-Heterotopie, Walker-Warburg-Syndrom, Alzheimer-Epilepsie, posttraumatischer Epilepsie, progressiver Myoklonus-Epilepsie, Reflex-Epilepsie, Rasmussen-Syndrom, Temporallappenepilepsie, limbischer Epilepsie, Status epilepticus, abdominaler Epilepsie, massivem bilateralem Myoklonus, katamenialer Epilepsie, Jackson-Anfällen, Unverricht-Lundborg-Krankheit, photosensitiver Epilepsie, Epilepsie mit nicht-genetischer Ätiologie, durch Gehirnerschütterung verursachter Epilepsie und durch Hirnverletzung verursachter Epilepsie.

6. Für PDE4B selektiver PDE4-Inhibitor zur Verwendung nach Anspruch 1 bis 5, wobei die Person an einer durch eine genetische Mutation verursachten Epilepsie leidet.

## Revendications

1. Inhibiteur de phosphodiestérase 4 (PDE4) pour utilisation dans un procédé de traitement de l'épilepsie, dans lequel le procédé comprend une administration à un individu souffrant d'épilepsie d'une quantité thérapeutiquement efficace de l'inhibiteur de PDE4, et dans lequel l'inhibiteur de PDE4 est sélectif pour PDE4B.

2. Inhibiteur de PDE4 qui est sélectif pour PDE4B pour utilisation selon la revendication 1, dans lequel l'inhibiteur de PDE4 est une petite molécule.

3. Inhibiteur de PDE4 qui est sélectif pour PDE4B pour utilisation selon la revendication 1 ou 2, dans lequel l'inhibiteur de PDE4 est un inhibiteur allostérique.

4. Inhibiteur de PDE4 qui est sélectif pour PDE4B pour utilisation selon l'une quelconque des revendications 1 à 3, dans lequel l'administration est par administration orale, parentérale, intranasale, intrathécale, intracrânienne ou transdermique.

5. Inhibiteur de PDE4 qui est sélectif pour PDE4B pour utilisation selon l'une quelconque des revendications 1 à 4, dans lequel l'individu présente une épilepsie choisie dans le groupe comprenant : épilepsie Rolandique bénigne, épilepsie du lobe frontal, spasmes infantiles, épilepsie myoclonique juvénile (JME), épilepsie d'absence juvénile, épilepsie d'absence d'enfance, pyknolepsie, convulsions fébriles, épilepsie myoclonique progressive de Lafora, syndrome de Lennox-Gastaut, syndrome de Landau-Kleffner, syndrome de Dravet (DS), épilepsie généralisée avec convulsions fébriles (GEFS+), épilepsie myoclonique sévère de l'enfance (SMEI), convulsions familiales néonatales bénignes (BFNC), syndrome de West, syndrome d'Ohtahara, encéphalopathie myoclonique précoce, épilepsie partielle migratoire, encéphalopathies épileptiques infantiles, complexe de sclérose tubéreuse (CST), dysplasie corticale focale, lissencéphalie de type I, syndrome de Miller-Dieker, syndrome d'Angelman, syndrome de l'X fragile, épilepsie dans les troubles du spectre autistique, hétérotopie sous-corticale, syndrome de Walker-Warburg, épilepsie de la maladie d'Alzheimer, épilepsie post-traumatique, épilepsie myoclonique progressive, épilepsie réflexe, syndrome de Rasmussen, épilepsie du lobe temporal, épilepsie limbique, épilepsie d'état, épilepsie abdominale, myoclonie bilatérale massive, épilepsie cataméniale, trouble convulsif Jacksonien, maladie d'Unverricht-Lundborg, épilepsie photosensible, épilepsie ayant une étiologie non génétique, épilepsie causée par une commotion cérébrale et épilepsie causée par une lésion cérébrale.

6. Inhibiteur de PDE4 qui est sélectif pour PDE4B pour utilisation selon l'une quelconque des revendications 1 à 5, dans lequel l'individu présente une épilepsie causée par une mutation génétique.
